# EUROPEAN PATENT APPLICATION

(11) **EP 4 413 998 A1**
(43) Date of publication of application: **14.08.2024**
(21) Application number: 22878590.3
(22) Date of filing: 06.10.2022
(51) Int. Cl.: A61K 39/395, A61K 9/08, A61K 39/00, A61K 47/10, A61M 5/28, A61P 1/04, A61P 1/14, A61P 37/06, C07K 16/28, C12N 15/13, G16H 70/40

(54) **METHOD FOR PREPARING PREFILLED SYRINGE FORMULATION**

(30) Priority: 08.10.2021 JP 2021166336; 17.03.2022 JP 2022042112
(71) Applicant: CHUGAI SEIYAKU KABUSHIKI KAISHA, Tokyo 115-8543 (JP)
(72) Inventor: ARAI, Kengo, Tokyo 115-8543 (JP); HIRAYAMA, Kazunori, Tokyo 115-8543 (JP); EGAMI, Kiichi, Tokyo 115-8543 (JP); FUKUDA, Masakazu, Tokyo 115-8543 (JP)
(74) Representative: Vossius & Partner Patentanwälte Rechtsanwälte mbB
(86) International application number: PCT/JP2022/037469
(87) International publication number: WO 2023/058723

(57) **Abstract**

An object of the present invention is to reduce formation of visually detectable particles in a formulation for injection in which a solution containing a protein is filled in a container. The present invention provides a method for determining a protein having a high risk of forming particles.

## Description

### TECHNICAL FIELD

The present invention relates to a pharmaceutical formulation that comprises a protein in a solution as an active ingredient, and is filled in a container.

### BACKGROUND ART

In recent years, various antibody formulations have been developed and put into practical use, but many antibody formulations are used as formulations for intravenous injection. Meanwhile, due to needs in actual medical practice, there are increasing demands for developing antibody-containing formulations as self-injectable formulations for subcutaneous injection. Especially, there is a high demand for developing a liquid formulation enclosed in a pre-filled syringe due to its convenience.

In design of an antibody-containing formulation for subcutaneous injection, it is essential that the concentration of an antibody in a liquid to be administered is high because while the amount of the antibody to be administered per dose is large (about 80 to 200 mg), the amount of an injection liquid is generally restricted in subcutaneous injection.

In recent years, a pre-filled syringe is being used in actual medical practice as a pre-filled syringe formulation for self-injection, which comprises a cylindrical injection syringe body filled in with a drug, a needle attached to the leading end of the injection syringe body, a detachably-attached syringe cap covering the needle, and a plunger that is inserted into the injection syringe body and is slidable in the axial direction of the injection syringe body.

In using the pre-filled syringe, the syringe cap is removed, the needle is inserted into an administration site, and then the plunger is moved forward with a plunger rod to excrete and administer a drug solution. Generally, in order to ensure slidability of the plunger, a lubricant of silicone oil or the like is applied to the inside wall of the pre-filled syringe and the plunger.

Formation of particles in an aqueous solution is a problem in an antibody-containing formulation. The particles to be formed are aggregates that are larger than multimers such as dimers and trimers, and known particles include sub-visible particles (SVPs), that is, microparticles with a particle size of 1.5 µm to less than 50 µm that are generally difficult to see with eyes, and visible particles (VPs, larger than 100 µm) that are visually detectable under standard illuminance (about 2,000 to 3,000 lx). A visual detection rate of visible particles in a pharmaceutical formulation varies greatly depending on an examiner, and under standard illuminance prescribed in the Japanese pharmacopoeia (about 2,000 to 3,000 lx), it is reported that detection sensitivity of particles with a particle size of 100 µm is about 40%, detection sensitivity of particles with a particle size of 150 µm is about 70%, and detection sensitivity of particles with a particle size of 200 µm is almost 100% (Non Patent Literature 1). Actually, particles with a smaller particle size of a minimum of about 40 µm can be visually detected by increasing illuminance for observing a pharmaceutical formulation or by increasing an observation time period. Herein, such particles in a range of from 40 µm to 100 µm are particularly referred to as particles visually detectable only under high illuminance. Besides, particles with a particle size of 40 µm or greater are particles visually detectable under high illuminance, and are referred to as visually detectable particles.

Generally, antibodies have a property of adsorbing to and aggregating on interfaces such as air-liquid interfaces and solid-liquid interfaces. The presence of such interfaces may contribute to the formation of the visually detectable particles described above. It is reported that application of mechanical stress to a syringe filled with an antibody solution results in marked increase of microparticles ascribable to the presence of an interface (Non Patent Literature 2). An air-liquid interface is formed in an antibody solution filled in a syringe due to presence of air bubbles, and a solid-liquid interface is formed when the solution contacts a plunger and a syringe barrel. Further, when silicone is applied to the plunger and the barrel of the pre-filled syringe, the antibody solution contacts the silicone on the solid phase surface and forms a new solid-liquid interface. Also, it is reported that proteins that adsorb to and aggregate on a solid-liquid interface are detached into the solution by the movement of air in the pre-filled syringe and appear as visible particles (Non Patent Literature 3).

An example of a method for reducing stress applied on various interfaces includes reduction of the amount of air bubbles in the pre-filled syringe. The amount of air that moves inside the pre-filled syringe can be reduced by reducing the amount of the air bubbles, and as a result, it is presumed that adsorption to an air-liquid interface or a solid-liquid interface and desorption of aggregates can be inhibited.

It has been reported that the amount of invisible particles and visible particles in a solution not containing a surfactant can be reduced in a pre-filled syringe containing a specific antibody by reducing the amount of air bubbles in the solution, but both of these literatures describe specific molecular theoretical results and evaluation results obtained under extremely unstable conditions (Patent Literatures 1 and 2).

Amino acid residues that are constituent elements of protein have different physical properties due to a difference in functional groups contained in side chains. Characteristics of physical properties of side chains can be roughly classified into two types depending on whether or not a functional group having a charge is included in the side chain, and how much the side chain is hydrophobic.

Those skilled in the art know that a three-dimensional structure model of a protein can be constructed in a computer with computational chemistry software by inputting the amino acid sequence information.

With respect to a charge among the physical properties of amino acid residues, a partial charge at an atomic level can be calculated by using a parameter designated as molecular force field. In Molecular Operating Environment (MOE; Chemical Computing Group Inc. (CCG)) used as the computational chemistry software, molecular force field designated as Amber 10: EHT is employed, and a partial charge of each atom constituting amino acid of protein is allocated by version ff10 of Amber force field (Non Patent Literature 4) having been continuously improved since publication in 1995. As an index of hydrophobicity of an amino acid residue, a hydrophobicity index in correlation with experimentally measurable octanol/water partition coefficient logP has been established in the 1990's, and an index developed by Crippen et al. is used in MOE (Non Patent Literature 5).

A method for detecting, at a predetermined level or higher, localization (patches) of a hydrophobic amino acid residue with a charge in the three-dimensional structure of protein based on these indexes of a charge and hydrophobicity of individual amino acid residues has been proposed in 1990's (Non Patent Literature 6), and it has been reported in 2018 that these can be detected with the above-described computational chemistry software MOE as a charged patch and a hydrophobic patch, respectively, and are correlated to some extent with experimental data obtained in drug discovery (Non Patent Literature 7).

Besides, as an example of application of the computational chemistry software MOE to medicament development, it has been reported that an area of a hydrophobic patch of an antibody calculated with MOE is correlated to some extent with a formation rate of visible particles (Non Patent Literature 8).

### CITATION LIST

### PATENT LITERATURE

[Patent Literature 1] Japanese Patent Laid-Open No. 2015-042638
[Patent Literature 2] International Publication No. WO 2017/184880

### NON PATENT LITERATURE

[Non Patent Literature 1] James A. Melchore, AAPS PharmSciTech; 2011; 12(1): 215-221
[Non Patent Literature 2] Torisu et al., J. Pharm. Sci. 106 (2017) 2966-2978
[Non Patent Literature 3] Gerhardt et al., J. Pharm. Sci. 103 (2014) 1601-1612
[Non Patent Literature 4] Cornell et al., J. Am. Chem. Soc. 1995, 117, 5179-5197
[Non Patent Literature 5] Wildman et al., J. Chem. Inf. Comput. Sci. 1999, 39, 868-873
[Non Patent Literature 6] Jones et al., J. Mol. Biol. 1997 272, 133-143
[Non Patent Literature 7] Jetha et al., MABS 2018, 10, 6, 890-900
[Non Patent Literature 8] Grapentin et al., J. Pharm. Sci. 109 (2020) 2393-2404

### SUMMARY OF INVENTION

### TECHNICAL PROBLEM

Nothing has been known about correlation between a parameter other than a hydrophobic patch based on a computation result of a three-dimensional structure model, and a risk of formation of visually detectable particles in a solution contained in a pre-filled syringe formulation. Besides, there is a demand for a better method for inhibiting particle formation.

The present inventors have found that it is difficult, in a biological medicament in which a large number of modifications have been caused in a molecule to increase deviation of hydrophobicity and charge, to completely inhibit formation of visually detectable particles even after addition of an appropriate amount of a surfactant.

### SOLUTION TO PROBLEM

Therefore, the present inventors have found the following: Regarding an antibody having a numerical value, calculated based on an area of a hydrophobic patch and an area of a charged patch, equal to or larger than a prescribed value, formation of visually detectable particles in a pre-filled syringe formulation, which cannot be sufficiently inhibited even after addition of a surfactant, can be greatly inhibited by reducing an air volume. The present specification encompasses the following disclosures of the invention.

[1-1] A method for determining, in a pharmaceutical formulation comprising a protein as an active ingredient in a solution, a protein having a high risk of forming particles in a solution, the method comprising: constructing a three-dimensional structure model of a protein based on an amino acid sequence of the protein by homology modeling or antibody modeling; specifying, in a surface of the obtained model, a portion where hydrophobic residues are accumulated in a cluster, and a portion where residues with a charge are accumulated in a cluster as a hydrophobic patch and a charged patch, respectively, and calculating areas of the patches; calculating a sum of areas of top 5 hydrophobic patches ranked according to the area (X ((angstrom)²)), and a total area of charged patches (Y ((angstrom)²)); and determining that a protein having a "X + Y × 1.5" value of 1,700 or greater is a protein having a high risk of forming particles in a solution, wherein the particles have a particle size of 40 µm or greater.

[1-2] The method according to [1-1], wherein the charge is a positive charge.

[1-3] The method according to [1-1], wherein the charge is a negative charge.

[1-4] The method according to any one of [1-1] to [1-3], wherein a protein having a "X + Y × 1.5" value of 2,000 or greater is determined as a protein having a high risk of forming particles in a solution.

[1-5] The method according to any one of [1-1] to [1-4], wherein Amber 10: EHT is used as molecular force field in the homology modeling or the antibody modeling.

[1-6] The method according to any one of [1-1] to [1-5], wherein the particles have a particle size larger than 100 µm.

[1-7] The method according to any one of [1-1] to [1-6], wherein the solution is an aqueous solution.

[1-8] The method according to any one of [1-1] to [1-7], wherein the protein is a monoclonal antibody, a fusion protein, a hormone, a cytokine, an enzyme, or a vaccine.

[1-9] The method according to any one of [1-1] to [1-8], wherein the protein is a monoclonal antibody.

[1-10] The method according to [1-9], wherein the monoclonal antibody is a monospecific antibody or a bispecific antibody.

[1-11] The method according to [1-9], wherein the monoclonal antibody is any one of IgG1, IgG2, and IgG4.

[1-12] The method according to any one of [1-1] to [1-11], wherein the constructing a three-dimensional structure model is performed by the antibody modeling.

[1-13] The method according to any one of [1-1] to [1-12], wherein the homology modeling or the antibody modeling is performed by Molecular Operating Environment (MOE) software.

[2-1] A method for determining, in a pharmaceutical formulation comprising a protein as an active ingredient in a solution, a protein having a high risk of forming particles in a solution, the method comprising: constructing a three-dimensional structure model of a protein based on an amino acid sequence of the protein by homology modeling or antibody modeling; specifying a portion on a surface of the obtained model corresponding to a cluster of residues with a charge as a charged patch, and calculating a total area of charged patches (Y ((angstrom)²)); and determining that a protein having a Y value of 600 or greater is a protein having a high risk of forming particles in a solution, wherein the particles have a particle size of 40 µm or greater.

[2-2] The method according to [2-1], wherein the charge is a positive charge.

[2-3] The method according to [2-1], wherein the charge is a negative charge.

[2-4] The method according to any one of [2-1] to [2-3], wherein a protein having a Y value of 700 or greater is determined as a protein having a high risk of forming particles in a solution.

[2-5] The method according to any one of [2-1] to [2-4], wherein Amber 10: EHT is used as molecular force field in the homology modeling or the antibody modeling.

[2-6] The method according to any one of [2-1] to [2-5], wherein the particles have a particle size larger than 100 µm.

[2-7] The method according to any one of [2-1] to [2-6], wherein the solution is an aqueous solution.

[2-8] The method according to any one of [2-1] to [2-7], wherein the protein is a monoclonal antibody, a fusion protein, a hormone, a cytokine, an enzyme, or a vaccine.

[2-9] The method according to any one of [2-1] to [2-8], wherein the protein is a monoclonal antibody.

[2-10] The method according to [2-9], wherein the monoclonal antibody is a monospecific antibody or a bispecific antibody.

[2-11] The method according to [2-9], wherein the monoclonal antibody is any one of IgG1, IgG2, and IgG4.

[2-12] The method according to any one of [2-1] to [2-11], wherein the constructing a three-dimensional structure model is performed by the antibody modeling.

[2-13] The method according to any one of [2-1] to [2-12], wherein the homology modeling or the antibody modeling is performed by Molecular Operating Environment (MOE) software.

[3-1] A method for reducing, in a formulation for injection in which a solution comprising a protein as an active ingredient is filled in a container, formation of particles in a solution, the method comprising: reducing a volume of air bubbles in the container to 40 µL or less, wherein the container is a syringe or a cartridge, and the protein is the protein determined, by the method according to any one of [1-1] to [1-13] and [2-1] to [2-13], to have a high risk of forming particles in a solution.

[3-2] The method according to [3-1], comprising reducing the volume of air bubbles in the container to 10 µL or less.

[3-3] The method according to [3-1] or [3-2], wherein the container is a syringe.

[3-4] The method according to [3-3], wherein the syringe is stoppered by a vacuum stopper placement method or a mechanical stopper placement method.

[3-5] The method according to any one of [3-1] to [3-4], wherein the particles have a particle size of 100 µm or greater.

[3-6] The method according to any one of [3-1] to [3-5], wherein the solution is an aqueous solution.

[3-7] The method according to any one of [3-1] to [3-6], wherein the protein is a monoclonal antibody, a fusion protein, a hormone, a cytokine, an enzyme, or a vaccine.

[3-8] The method according to any one of [3-1] to [3-7], wherein the protein is a monoclonal antibody.

[3-9] The method according to [3-8], wherein the monoclonal antibody is a monospecific antibody or a bispecific antibody.

[3-10] The method according to [3-8], wherein the monoclonal antibody is any one of IgG1, IgG2, and IgG4.

[3-11] The method according to [3-8], wherein the monoclonal antibody is an antibody having an H-chain of SEQ ID NOs: 3 and 4 and an L-chain of SEQ ID NO: 5, or an antibody having an H-chain of SEQ ID NO: 6 and an L-chain of SEQ ID NO: 7.

[3-12] The method according to [3-8], wherein the monoclonal antibody is an antibody having a combination of an H-chain of SEQ ID NO: 8 and an L-chain of SEQ ID NO: 9 and a combination of an H-chain of SEQ ID NO: 11 and an L-chain of SEQ ID NO: 10.

[4-1] A method for preparing a formulation for injection in which a solution comprising a protein as an active ingredient is filled in a container, comprising filling the solution in the container in such a manner as to have a volume of air bubbles of 40 µL or less in the container of the formulation for injection to be obtained, wherein the container is a syringe or a cartridge, and the protein is the protein determined, by the method according to any one of [1-1] to [1-13] and [2-1] to [2-13], to have a high risk of forming particles in a solution.

[4-2] The method according to [4-1], comprising filling the solution in the container in such a manner as to have a volume of air bubbles of 10 µL or less in the container of the formulation for injection to be obtained.

[4-3] The method according to [4-1] or [4-2], wherein the container is a syringe.

[4-4] The method according to [4-3], wherein the container is stoppered by a vacuum stopper placement method or a mechanical stopper placement method in filling the solution in the container.

[4-5] The method according to any one of [4-1] to [4-4], wherein the particles have a particle size of 100 µm or greater.

[4-6] The method according to any one of [4-1] to [4-5], wherein the solution is an aqueous solution.

[4-7] The method according to any one of [4-1] to [4-6], wherein the protein is a monoclonal antibody.

[4-8] The method according to [4-7], wherein the monoclonal antibody is a monospecific antibody or a bispecific antibody.

[4-9] The method according to [4-7], wherein the monoclonal antibody is any one of IgG1, IgG2, and IgG4.

[4-10] The method according to [4-7], wherein the monoclonal antibody is an antibody having an H-chain of SEQ ID NOs: 3 and 4 and an L-chain of SEQ ID NO: 5, or an antibody having an H-chain of SEQ ID NO: 6 and an L-chain of SEQ ID NO: 7.

[4-11] The method according to [4-7], wherein the monoclonal antibody is an antibody having a combination of an H-chain of SEQ ID NO: 8 and an L-chain of SEQ ID NO: 9 and a combination of an H-chain of SEQ ID NO: 11 and an L-chain of SEQ ID NO: 10.

[5-1] A formulation for injection in which a solution comprising a protein as an active ingredient is filled in a container, wherein the protein is the protein determined, by the method according to any one of [1-1] to [1-13] and [2-1] to [2-13], to have a high risk of forming particles in a solution, the container is a syringe or a cartridge, and a volume of air bubbles in the container is 40 µL or less.

[5-2] The formulation for injection according to [5-1], wherein the volume of air bubbles in the container is 10 µL or less.

[5-3] The formulation for injection according to [5-1] or [5-2], wherein the container is a syringe.

[5-4] The formulation for injection according to any one of [5-1] to [5-3], wherein a concentration of the protein in the solution is 0.1 mg/mL or more.

[5-5] The formulation for injection according to any one of [5-1] to [5-4], wherein a concentration of the protein in the solution is in a range of 0.1 to 300 mg/mL.

[5-6] The formulation for injection according to any one of [5-1] to [5-5], wherein a concentration of the protein in the solution is in a range of 1 to 200 mg/mL.

[5-7] The formulation for injection according to any one of [5-1] to [5-6], wherein an amount of the solution contained in a 1 mL syringe is in a range of 0.1 to 1.2 mL, or an amount of the solution contained in a 2.25 mL syringe is in a range of 0.1 to 2.5 mL.

[5-8] The formulation for injection according to any one of [5-1] to [5-7], wherein an amount of the solution contained in a 1 mL syringe is in a range of 0.2 to 1.1 mL, or an amount of the solution contained in a 2.25 mL syringe is in a range of 0.3 to 2.3 mL.

[5-9] The formulation for injection according to any one of [5-1] to [5-8], wherein the formulation for injection in which a solution comprising a protein as an active ingredient is filled in a container comprises a syringe or a cartridge holding a pharmaceutical formulation therein, and a stopper, and the syringe or the cartridge is made of glass or a cycloolefin-based resin.

[5-10] The formulation for injection according to [5-9], wherein the cycloolefin-based resin is a cycloolefin polymer (COP) or a cycloolefin copolymer (COC).

[5-11] The formulation for injection according to any one of [5-1] to [5-10], wherein the particles have a particle size of 100 µm or greater.

[5-12] The formulation for injection according to any one of [5-1] to [5-11], wherein the solution is an aqueous solution.

[5-13] The formulation for injection according to any one of [5-1] to [5-12], wherein the protein is a monoclonal antibody.

[5-14] The formulation for injection according to [5-13], wherein the monoclonal antibody is a monospecific antibody or a bispecific antibody.

[5-15] The formulation for injection according to [5-13], wherein the monoclonal antibody is any one of IgG1, IgG2, and IgG4.

[5-16] The formulation for injection according to [5-13], wherein the monoclonal antibody is an antibody having an H-chain of SEQ ID NOs: 3 and 4 and an L-chain of SEQ ID NO: 5, or an antibody having an H-chain of SEQ ID NO: 6 and an L-chain of SEQ ID NO: 7.

[5-17] The formulation for injection according to any one of [5-1] to [5-16], wherein the solution comprises one or more pharmaceutically acceptable excipients including a sugar, a sugar alcohol, a buffer, a preservative, a carrier, an antioxidant, a chelating agent, a natural polymer, a synthetic polymer, a cryoprotective agent, a surfactant, an extending agent, and a stabilizing agent, or a combination thereof.

[5-18] The formulation for injection according to [5-17], wherein the surfactant is polysorbate, poloxamer 188, sodium lauryl sulfate, polyol, poly(ethylene glycol), glycerol, propylene glycol, or poly(vinyl alcohol).

[5-19] The formulation for injection according to [5-17] or [5-18], wherein the surfactant is polysorbate or poloxamer 188.

[5-20] The formulation for injection according to any one of [5-17] to [5-19], wherein a concentration of the surfactant in the solution is 0.01 mg/mL or more.

[5-21] The formulation for injection according to any one of [5-17] to [5-20], wherein a concentration of the surfactant in the solution is in a range of 0.01 to 5 mg/mL.

[5-22] The formulation for injection according to [5-17] to [5-21], wherein a concentration of the surfactant in the solution is in a range of 0.25 to 0.75 mg/mL.

[5-23] The formulation for injection according to any one of [5-1] to [5-22], wherein a pH of the solution is in a range of 4.5 to 7.5.

[5-24] The formulation for injection according to any one of [5-1] to [5-23], wherein a pH of the solution is in a range of 5.0 to 7.0.

[5-25] The formulation for injection according to [5-1] to [5-24], wherein a pH of the solution is in a range of 5.5 to 6.5.

[5-26] The formulation for injection according to any one of [5-1] to [5-25], wherein an average number of particles in the formulation for injection after storage for 3 months with drop stress applied during the storage at 25°C is reduced as compared with that in employing a condition that the volume of air bubbles in the formulation for injection is 120 µL.

[5-27] The formulation for injection according to any one of [5-1] to [5-25], wherein an average number of particles in the formulation for injection comprising 0.01 mg/mL of a surfactant after storage for 1 day at 5°C is reduced as compared with that in employing a condition that the volume of air bubbles in the formulation for injection is 120 µL.

[5-28] The formulation for injection according to any one of [5-13], [5-14] or [5-17] to [5-26], wherein the monoclonal antibody is an antibody having a combination of an H-chain of SEQ ID NO: 8 and an L-chain of SEQ ID NO: 9 and a combination of an H-chain of SEQ ID NO: 11 and an L-chain of SEQ ID NO: 10.

[6-1] A system for determining, in a pharmaceutical formulation comprising a protein as an active ingredient in a solution, a protein having a high risk of forming particles in a solution, the system comprising: means for constructing a three-dimensional structure model of a protein based on an amino acid sequence of the protein by homology modeling or antibody modeling; means for specifying, in a surface of the obtained model, a portion where hydrophobic residues are accumulated in a cluster, and a portion where residues with a charge are accumulated in a cluster as a hydrophobic patch and a charged patch, respectively, and calculating areas of the patches; means for calculating a sum of areas of top 5 hydrophobic patches ranked according to the area (X ((angstrom)²)), and a total area of charged patches (Y ((angstrom)²)); and means for determining that a protein having a "X + Y × 1.5" value of 1,700 or greater is a protein having a high risk of forming particles in a solution, wherein the particles have a particle size of 40 µm or greater.

[6-2] The system according to [6-1], wherein the charge is a positive charge.

[6-3] The system according to [6-1], wherein the charge is a negative charge.

[6-4] The system according to any one of [6-1] to [6-3], wherein a protein having a "X + Y × 1.5" value of 2,000 or greater is determined as a protein having a high risk of forming particles in a solution.

[6-5] The system according to any one of [6-1] to [6-4], wherein Amber 10: EHT is used as molecular force field in the homology modeling or the antibody modeling.

[6-6] The system according to any one of [6-1] to [6-5], wherein the particles have a particle size larger than 100 µm.

[6-7] The system according to any one of [6-1] to [6-6], wherein the solution is an aqueous solution.

[6-8] The system according to any one of [6-1] to [6-7], wherein the protein is a monoclonal antibody, a fusion protein, a hormone, a cytokine, an enzyme, or a vaccine.

[6-9] The system according to any one of [6-1] to [6-8], wherein the protein is a monoclonal antibody.

[6-10] The system according to [6-9], wherein the monoclonal antibody is a monospecific antibody or a bispecific antibody.

[6-11] The system according to [6-9], wherein the monoclonal antibody is any one of IgG1, IgG2, and IgG4.

[6-12] The system according to any one of [6-1] to [6-11], wherein the constructing a three-dimensional structure model is performed by the antibody modeling.

[6-13] A program causing a computer to operate the respective means of the system according to any one of [6-1] to [6-12].

[6-14] A storage medium storing the program according to [6-13].

[6-15] An apparatus for determining, in a pharmaceutical formulation comprising a protein as an active ingredient in a solution, a protein having a high risk of forming particles in a solution, comprising the program according to [6-13] installed therein.

[7-1] A system for determining, in a pharmaceutical formulation comprising a protein as an active ingredient in a solution, a protein having a high risk of forming particles in a solution, the system comprising: means for constructing a three-dimensional structure model of a protein based on an amino acid sequence of the protein by homology modeling or antibody modeling; means for specifying a portion on a surface of the obtained model corresponding to a cluster of residues with a charge as a charged patch, and calculating a total area of charged patches (Y ((angstrom)²)); and means for determining that a protein having a Y value of 600 or greater is a protein having a high risk of forming particles in a solution, wherein the particles have a particle size of 40 µm or greater.

[7-2] The system according to [7-1], wherein the charge is a positive charge.

[7-3] The system according to [7-1], wherein the charge is a negative charge.

[7-4] The system according to any one of [7-1] to [7-3], wherein a protein having a Y value of 700 or greater is determined as a protein having a high risk of forming particles in a solution.

[7-5] The system according to any one of [7-1] to [7-4], wherein Amber 10: EHT is used as molecular force field in the homology modeling or the antibody modeling.

[7-6] The system according to any one of [7-1] to [7-5], wherein the particles have a particle size larger than 100 µm.

[7-7] The system according to any one of [7-1] to [7-6], wherein the solution is an aqueous solution.

[7-8] The system according to any one of [7-1] to [7-7], wherein the protein is a monoclonal antibody, a fusion protein, a hormone, a cytokine, an enzyme, or a vaccine.

[7-9] The system according to any one of [7-1] to [7-8], wherein the protein is a monoclonal antibody.

[7-10] The system according to [7-9], wherein the monoclonal antibody is a monospecific antibody or a bispecific antibody.

[7-11] The system according to [7-9], wherein the monoclonal antibody is any one of IgG1, IgG2, and IgG4.

[7-12] The system according to any one of [7-1] to [7-11], wherein the constructing a three-dimensional structure model is performed by the antibody modeling.

[7-13] A program causing a computer to operate the respective means of the system according to any one of [7-1] to [7-12].

[7-14] A storage medium storing the program according to [7-13].

[7-15] An apparatus for determining, in a pharmaceutical formulation comprising a protein as an active ingredient in a solution, a protein having a high risk of forming particles in a solution, comprising the program according to [7-13] installed therein.

### ADVANTAGEOUS EFFECTS OF INVENTION

In one aspect of the present invention, it is possible to determine a protein having a high risk of forming visually detectable particles in a solution of a pre-filled syringe formulation. In another aspect of the present invention, a pre-filled syringe formulation in which formation of visually detectable particles is maximally inhibited can be provided.

### BRIEF DESCRIPTION OF DRAWINGS

Figure 1 illustrates photographs of syringes containing air bubbles in a volume of 120 µL (a), 40 µL (b), and 10 µL (c).
Figure 2 is a schematic diagram of a cardboard box used in a drop test.
Figure 3 is an exemplified configuration diagram of a syringe.
Figure 4 is a histogram of sizes of visually detectable proteinaceous particles of a protein identified in Example 3.
Figure 5 illustrates a process flow in executing a program for an apparatus for determining a protein having a high risk of forming particles in a solution based on a hydrophobic patch and a charged patch.
Figure 6 illustrates a process flow in executing a program for an apparatus for determining a protein having a high risk of forming particles in a solution based on a charged patch.
Figure 7 is a schematic configuration diagram of an apparatus for determining a protein having a high risk of forming particles in a solution.

### DESCRIPTION OF EMBODIMENTS

### (1) Determination of risk of forming visually detectable particles

Herein, a visually detectable particle refers to a particle that is visually detectable under high illuminance and has a particle size of 40 µm or greater. Among them, particles that are visually detectable under standard illuminance as prescribed in the Japanese pharmacopoeia (about 2,000 to 3,000 lx) are designated as "visible particles" or "insoluble visible particles". Generally, visible particles have a particle size greater than 100 µm (Non Patent Literature 1). Particles that are smaller in size than visible particles, and cannot be seen with eyes with standard illuminance as prescribed in the Japanese pharmacopoeia (about 2,000 to 3,000 lx) but can be visually detected by increasing illuminance or by increasing an observation time period are "particles visually detectable only under high illuminance", and have a particle size of 40 µm to 100 µm. Visible particles can be confirmed by visual inspection with naked eyes for 5 seconds or longer under illumination at standard illuminance (about 2,000 to 3,000 lx), by slowly rotating or inverting the container in front of a black background or a white background. Particles visually detectable only under high illuminance can be confirmed by visual inspection with naked eyes for 30 seconds or longer under illumination at high illuminance (6,000 lx or greater) by slowly rotating or inverting the container in front of a black background. Visible particles can also be confirmed with inspection under high illuminance. Particles except for those generated from protein molecules in a solution are not considered as "visually detectable particles" regardless of the size. Whether visually detectable particles are derived from protein molecules can be confirmed by Raman microspectroscopic measurement. The only content contained as a protein in the solution is an active pharmaceutical ingredient (API), and visually detectable particles are generated from API. The particle size and number of visually detectable particles can be determined by a light obscuration particle count method, a microscopic particle count method, a flow cytometric particle image analysis method, visual inspection, and infrared microspectroscopy (infrared spectroscopy ;IR) or Raman microspectroscopic measurement performed after isolation of particles, and are measured preferably by a combination of visual inspection and infrared microspectroscopy or Raman microspectroscopic measurement.

Herein, the term "high risk of forming particles" in a solution of a pharmaceutical formulation implies that protein molecules in the solution easily aggregate and form visually detectable particles. Examples of proteins having a high risk of forming particles include proteins that can form visually detectable particles even after addition of an appropriate amount of a surfactant.

Herein, the term "air bubble" refers to a space between a liquid in a container and the wall of the container, or gas present in the liquid. The air bubble is of a size that can be seen with naked eyes or by light-microscopic examination. The term "in the container" includes, in the case of a pre-filled syringe with a needle, the entire space stoppered with a rigid needle shield (RNS) and a stopper, and more specifically includes a space inside the needle and inside the barrel. When the container is in a vertical position, an air bubble does not extend across the diameter of the container and not all but part of the liquid touches the bottom surface of a container closure (such as a stopper). In one embodiment, an air bubble is spherical. In another embodiment, an air bubble is not spherical. In such an embodiment, the air bubble has an egg-shape.

In one aspect of the present invention, the volume of air bubbles may be 120 µL or less, 110 µL or less, 100 µL or less, 90 µL or less, 80 µL or less, 70 µL or less, 60 µL or less, 50 µL or less, 40 µL or less, 30 µL or less, 20 µL or less, or 10 µL or less. In one embodiment of the present invention, the volume of air bubbles is determined as the volume of air bubbles obtained when all the bubbles in the container are unified. The volume of air bubbles may be determined by a method of "actually measuring the volume of air bubbles by extruding from the needle tip in the order of the gas and the solution into an appropriate container with scales such as a pipette already containing a solution", "obtaining an image and calculating the volume of air bubbles based on the area of air bubble portions", or "calculating the volume of air bubbles based on the height of a bubble portion on the basis of already-known information on the barrel internal diameter".

In one aspect of the present invention, "homology modeling" is employed for determining a protein having a high risk of forming particles in a solution. "Homology modeling" is a method for estimating a three-dimensional structure of a protein having a specific sequence based on similarity in sequence to one or more proteins having known three-dimensional structures. Usually, homology modeling for a specific amino acid sequence includes the following steps of: 1) specifying a homolog having a known structure in the Protein Data Bank, 2) arranging a target sequence in correspondence to a template structure, 3) constructing a model based on such alignment, and 4) evaluating and refining the model (Xiang, Curr Protein Pept Sci. 2006 June; 7(3): 217-227). Examples of software for estimating a three-dimensional structure equipped with homology modeling function include, but are not limited to, Molecular Operating Environment (MOE; Chemical Computing Group Inc. (CCG) (Canada), Web Antibody Modelling (WAM; http://antibody.bath.ac.uk), Rosetta (https://www.rosettacommons.org/software), Prime (Schrodinger), MODELLER (Eswar, et al., Comparative Protein Structure Modeling With MODELLER, Current Protocols in Bioinformatics, John Wiley & Sons, Inc., Supplement 15, 5.6.1-5.6.30, 200), SEGMOD/ENCAD (Levitt M. J Mol Biol 1992; 226: 507-533), SWISS-MODEL (Schwede T., Kopp J., Guex N., Peitsch M. C. Nucleic Acids Research 2003; 31: 3381-3385.), 3D-JIGSAW (Bates et al., Proteins: Structure, Function and Genetics, Suppl 2001; 5: 39-46), NEST (Xiang, Curr Protein Pept Sci. 2006 June; 7(3): 217-227), and BUILDER (Koehl and Delarue, Curr Opin Struct Biol 1996; 6(2): 222-226). A preferred example of the software includes MOE.

In one aspect of the present invention, the term "antibody modeling" refers to a function for estimating a three-dimensional structure, and database specialized in monoclonal antibodies. In antibody modeling, the entire structure can be assembled based on a structure of a fragment. For example, an antibody Fab fragment can be added to an Fc fragment crystal structure, and a Fab fragment can be formed as an estimated protein structure and added to an Fc fragment crystal structure. For example, functions provided in MOE can be used to carry out antibody modeling.

In one aspect of the present invention, the term "patch" refers to a surface region of a cluster of residues representing a specific physicochemical property in a three-dimensional structure of a protein or an antibody. Examples of the patch include a hydrophobic patch and a charged patch. A hydrophobic patch is a surface region of a portion where hydrophobic residues are accumulated in a cluster. The portion where hydrophobic residues are accumulated in a cluster can also include residues other than hydrophobic residues. A charged patch is a surface region of a portion where residues with a charge are accumulated in a cluster. The portion where residues with a charge are accumulated in a cluster can also include residues without a charge.

In one aspect of the present invention, a feature amount concerning a patch area of a protein can be calculated using Protein properties function of MOE. With respect to the patch area of a specific protein surface, when a sum of areas of top 5 hydrophobic patches ranked according to the area of hydrophobic patches is set as X ((angstrom)²), and a total area of charged patches is set as Y ((angstrom)²), a risk of the protein of forming particles can be determined based on a "X + Y × 1.5" value. In one embodiment, a protein having a "X + Y × 1.5" value of 1,700 or greater can be determined as a protein having a high risk of forming particles. In another embodiment, a protein having a "X + Y × 1.5" value of 2,000 or greater, 2,500 or greater, 3,000 or greater, 3,500 or greater, or 4,000 or greater can be determined as a protein having a high risk of forming particles.

In one aspect of the present invention, the feature amount concerning a patch area of a protein can be calculated using Protein properties function of MOE, and with respect to the patch area of a specific protein surface, a risk of the protein of forming particles can be determined based on a total area of charged patches Y ((angstrom)²). In one embodiment, a protein having a Y value of 600 or greater is determined as a protein having a high risk of forming particles. In another embodiment, a protein having a Y value of 700 or greater, 800 or greater, 900 or greater, 1,000 or greater, 1,500 or greater, 2,000 or greater, 2,500 or greater, 3,000 or greater, or 4,000 or greater is determined as a protein having a high risk of forming particles.

Herein, the term ranking according to the area of hydrophobic patches refers to a list of hydrophobic patches identified on a protein surface and arranged in the order of the area, and each hydrophobic patch means a hydrophobic patch consisting of a cluster of hydrophobic residues, independently present on the protein surface and having a certain size. A sum of areas of top 5 hydrophobic patches in the ranking ((angstrom)²) is calculated as X. Herein, a sum of the areas of top 5 hydrophobic patches refers to a total value of the areas of the top 5 hydrophobic patches. However, if there are 4 or less hydrophobic patches in a molecule, the sum refers to a total value of the areas of all the hydrophobic patches actually present.

A total area of charged patches means the sum of the areas ((angstrom)²) of all positively or negatively charged patches present on the protein surface.

In one aspect of the present invention, the term "molecular force field" is parameterization, in the form of a function, of force applied to each atom present in a molecule. In molecular mechanics calculation and molecular dynamics calculation based on molecular force field, inter-atomic force is expressed as numeric values of potential function determined according to the types of atoms and mode of binding, with parameters representing inter-atomic bonds (such as a bond distance and a bond angle) as variables. In molecular mechanics calculation and molecular dynamics calculation based on molecular force field, inter-atomic force is expressed as numeric values of potential function determined according to the types of atoms and mode of binding, with parameters representing inter-atomic bonds (such as a bond distance and a bond angle) as variables.

In one aspect of the present invention, the molecular force field that can be used is not particularly limited but can be appropriately selected depending on purpose, and examples include Amber molecular force field, CHARMm molecular force field, and OPLS molecular force field. Examples of Amber molecular force field include Amber 10/14: EHT, Amber ff99SB-ILDN, and Amber 12SB. An example of CHARMm molecular force field includes CHARMm 36. Among these, Amber 10: EHT is preferable when MOE is used.

### (2) Reduction of particle formation

In one aspect of the present invention, the term "reducing formation of particles" refers to adjusting the volume of air bubbles so as not to form visually detectable particles or to reduce the number of formed particles in a solution of a pharmaceutical formulation in which visually detectable particles are formed under a given condition. Reduction of formation of visually detectable particles can be confirmed by counting the number of particles before and after adjusting the volume of air bubbles. The size and number of particles can be determined by a light obscuration particle count method, a microscopic particle count method, a flow cytometric particle image analysis method, visual inspection, and infrared microspectroscopy (infrared spectroscopy ;IR) or Raman microspectroscopic measurement after isolation of particles, and are measured preferably by a combination of visual inspection and infrared microspectroscopy or Raman microspectroscopic measurement.

### (3) Pharmaceutical formulation

In one aspect of the present invention, a pharmaceutical formulation is a solution containing a protein as an active ingredient. The pharmaceutical formulation may be a formulation for injection.

In one aspect of the present invention, a formulation for injection is a pharmaceutical formulation that is filled in a container for injection to be administered by injection, and contains a protein as an active ingredient in a solution.

In one aspect of the present invention, the term "formulation for injection to be obtained" refers to a formulation for injection obtained as a final product after adjusting the air volume.

In one aspect of the present invention, the pharmaceutical formulation is stored without freezing the solution in the container at -30°C to 25°C, preferably from the freezing point of the solution to 25°C, more preferable at 1°C to 10°C, more preferably at 2°C to 8°C, and even more preferably at 5°C. The storage is carried out for 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 48 hours, 60 hours, 72 hours, 84 hours, or 96 hours. The storage is carried out for at least 24 hours, at least 2 days, at least 3 days, at least 4 days, at least 10 days, at least 20 days, at least 30 days, at least 40 days, at least 50 days, at least 60 days, at least 1 month, at least 2 months, at least 3 months, at least 4 months, at least 5 months, at least 6 months, at least 7 months, at least 8 months, at least 9 months, at least 10 months, at least 11 months, or at least 12 months.

In one aspect of the present invention, the protein used in a liquid formulation encompasses, but is not limited to, an antibody, a fusion protein, an enzyme, a hormone, a cytokine, and a vaccine. More specifically, the protein encompasses a monoclonal antibody, granulocyte colony-stimulating factor (G-CSF), granulocyte macrophage colony-stimulating factor (GM-CSF), erythropoietin (EPO), interferon, interleukins such as IL-1 or IL-6, tissue plasminogen activator (TPA), thrombopoietin, urokinase, serum albumin, blood coagulation factor VIII, leptin, stem cell factor (SCF), and the like.

In one aspect of the present invention, the protein used in the pharmaceutical formulation has substantially the same biological activity as a bioactive protein of a mammal, in particular of a human, and encompasses proteins derived from nature and those obtained by genetic engineering. Proteins obtained by genetic engineering include those having the same amino acid sequence as a native protein, or those obtained by deleting, substituting, or adding one or more amino acid sequences and having the above-described biological activity.

In one aspect of the present invention, a concentration of the protein in the solution may be 0.1 mg/mL or more, within a range of 0.1 to 300 mg/mL, or within a range of 1 to 200 mg/mL.

In one aspect of the present invention, an antibody to be used is not particularly limited as long as it binds to a desired antigen, may be a polyclonal antibody or a monoclonal antibody, and is preferably a monoclonal antibody because a homogeneous antibody can be thus stably produced. In one aspect of the present invention, the antibody to be used may be a monospecific antibody or a bispecific antibody, or may be a multispecific antibody having three or more antigen-recognizing sites in a molecule.

In one aspect of the present invention, the monoclonal antibody to be used encompasses not only a monoclonal antibody derived from an animal, such as a human, a mouse, a rat, a hamster, a rabbit, a sheep, a camel, or a monkey, but also a recombinant antibody obtained by artificial modification, such as a chimeric antibody, a humanized antibody, or a bispecific antibody. Furthermore, a recombinant antibody obtained by artificial modification of a constant region or the like of an antibody for modifying physical properties of an antibody molecule (specifically, modification of an isoelectric point (pI), modification of affinity of Fc receptor, and the like) for purposes of improving retention in blood and pharmacokinetics is also encompassed.

In one aspect of the present invention, the immunoglobulin class of the antibody to be used is not particularly limited, but may be any of classes including IgG such as IgG1, IgG2, IgG3, and IgG4, and IgA, IgD, IgE, and IgM, among which IgG is preferred, and IgG1, IgG2 and IgG4 are particularly preferred.

Further, in one aspect of the present invention, the antibody to be used encompasses not only an antibody including a constant region and a variable region (full-length antibody) but also an antibody fragment, such as Fv, Fab, and F(ab)₂, and a low-molecular-weight antibody like a bispecific antibody such as one- or two-associated single chain Fv (scF, sc(Fv)₂) having a variable region of the antibody bound with a linker such as a peptide linker, or a scFv dimer, and a full-length antibody is preferred.

In one aspect of the present invention, the antibody to be used can be produced by a known method. A hybridoma used for producing a monoclonal antibody can be produced as follows basically by known techniques. Specifically, a desired antigen or a cell expressing a desired antigen used as a sensitizing antigen is immunized by a usual immunization method, and the resultant immune cell is fused with a known parent cell by a usual cell fusion method, the resultant is subjected to a usual screening method for screening a monoclonal antibody producing cell (hybridoma), and thus, the hybridoma can be produced. The production of a hybridoma can be carried out in accordance with, for example, the method of Milstein et al., (Kohler, G. and Milstein, C., Methods Enzymol. (1981) 73: 3-46) or the like. If immunogenicity of the antigen is low, the antigen may be bound to a macromolecule having immunogenicity, such as albumin, before the immunization.

Alternatively, a recombinant antibody obtained by cloning an antibody gene from a hybridoma, and incorporating the gene into an appropriate vector to be introduced into a host by genetic engineering techniques can be used (see, for example, Carl, A. K. Borrebaeck, James, W. Larrick, THERAPEUTIC MONOCLONAL ANTIBODIES, Published in the United Kingdom by MACMILLAN PUBLISHERS LTD., 1990). Specifically, cDNA of a variable region (V region) of an antibody is synthesized from mRNA of the hybridoma with reverse transcriptase. DNA encoding the V region of the target antibody thus obtained is linked to DNA encoding a desired antibody constant region (C region), and the resultant is incorporated into an expression vector. Alternatively, DNA encoding the V region of the antibody may be incorporated into an expression vector containing DNA of an antibody C region. The resultant is incorporated into the expression vector so as to express under control of an expression control region, such as an enhancer or a promoter. Next, a host cell is transformed with the resultant expression vector, and thus, the antibody can be expressed.

In one aspect of the present invention, a recombinant antibody obtained by artificial modification for purpose of reducing heteroantigenicity against human, such as a chimeric antibody or a humanized antibody, can be used. Such a modified antibody can be produced by a known method. A chimeric antibody is an antibody containing heavy-chain and light-chain variable regions of an antibody of a mammal other than a human, for example, a mouse antibody, and heavy-chain and light-chain constant regions of a human antibody, and can be obtained by linking DNA encoding a variable region of a mouse antibody to DNA encoding a constant region of a human antibody, incorporating the resultant into an expression vector, and introducing the vector into a host to produce the antibody.

A humanized antibody is designated also as a reshaped human antibody, and is obtained by transplanting a complementary determining region (CDR) of an antibody of a mammal other than a human, for example, a mouse antibody, into a complementary determining region of a human antibody, and a general genetic engineering method for such an antibody is also known. Specifically, a DNA sequence designed to link CDR of a mouse antibody to a framework region (FR) of a human antibody is synthesized by PCR method from several oligonucleotides produced to have overlapping portions at the ends. The thus obtained DNA is linked to DNA encoding a human antibody constant region, the resultant is subsequently incorporated into an expression vector, and the resultant vector is introduced into a host to produce the antibody (see European Patent Application No. 239400, and WO 96/02576). As the FR of a human antibody to be linked via CDR, one having a complementary determining region forming a good antigen-binding site is selected. If necessary, an amino acid in a framework region of a variable region of an antibody may be substituted so as to cause the complementary determining region of the reshaped human antibody to form a suitable antigen-binding site (Sato, K. et al., Cancer Res. (1993) 53, 851-856).

As techniques for substituting an amino acid of an antibody for improving activity, physical properties, pharmacokinetics, safety and the like of the antibody, for example, the following techniques are known, and in one aspect of the present invention, the antibody to be used encompasses such an antibody having substitution (including deletion and addition) of an amino acid.

As techniques for amino acid substitution in a variable region of an IgG antibody, not only humanization (Tsurushita N., Hinton P.R., Kumar S., Design of humanized antibodies: from anti-Tac to Zenapax., Methods, 2005 May; 36 (1): 69-83) but also affinity maturation by amino acid substitution in a complementary determining region (CDR) for enhancing binding activity (Rajpal A., Beyaz N., Haber L., Cappuccilli G., Yee H., Bhatt R.R., Takeuchi T., Lerner R.A., Crea R., A general method for greatly improving the affinity of antibodies by using combinatorial libraries, Proc Natl Acad Sci USA, 2005 Jun 14; 102 (24): 8466-71), and improvement of physicochemical stability by amino acid substitution in framework (FR) (Ewert S., Honegger A., Pluckthun A., Stability improvement of antibodies for extracellular and intracellular applications: CDR grafting to stable frameworks and structure-based framework engineering, Methods, 2004 Oct; 34(2): 184-99. Review) have been reported. As techniques for amino acid substitution in Fc region of an IgG antibody, techniques for enhancing antibody dependent cellular cytotoxicity (ADCC) or complement dependent cellular cytotoxicity (CDC) are known (Kim S.J., Park Y., Hong H.J., Antibody engineering for the development of therapeutic antibodies, Mol Cells, 2005 Aug 31; 20(1): 17-29 Review.). In addition, techniques for amino acid substitution in Fc by not only enhancing such effector function but also improving half-life in blood of an antibody have been reported (Hinton P.R., Xiong J.M., Johlfs M.G., Tang M.T., Keller S., Tsurushita N., An engineered human IgG1 antibody with longer serum half-life, J Immunol. 2006 Jan 1; 176(1): 346-56, Ghetie V., Popov S., Borvak J., Radu C., Matesoi D., Medesan C., Ober R.J., Ward E.S., Increasing the serum persistence of an IgG fragment by random mutagenesis, Nat Biotechnol. 1997 Jul; 15(7): 637-40.). Furthermore, various techniques for amino acid substitution in a constant region for purposes of improving physical properties of an antibody are known (WO 09/41613).

Besides, there are known methods for obtaining a human antibody. For example, human lymphocyte is sensitized with a desired antigen or a cell expressing a desired antigen *in vitro,* and the thus sensitized lymphocyte is fused to a human myeloma cell, for example, U266, and thus, a desired human antibody having a binding activity to an antigen can be obtained (see Japanese Patent Publication No. 1-59878). Furthermore, when a transgenic animal having all repertoires of human antibody genes is immunized with an antigen, a desired human antibody can be obtained (see WO 93/12227, WO 92/03918, WO 94/02602, WO 94/25585, WO 96/34096, and WO 96/33735). Besides, a technique for obtaining a human antibody by panning using a human antibody library is also known. For example, a variable region of a human antibody is expressed as a single chain antibody (scFv) on the surface of a phage by a phage display method, and thus, a phage binding to an antigen can be selected. When the gene of the selected phage is analyzed, a DNA sequence encoding the variable region of a human antibody binding to the antigen can be determined. When the DNA sequence of scFv binding to the antigen is clarified, an appropriate expression vector containing the sequence can be produced to obtain a human antibody. These methods are already known, and can be executed with the reference to WO 92/01047, WO 92/20791, WO 93/06213, WO 93/11236, WO 93/19172, WO 95/01438, and WO 95/15388. In one aspect of the present invention, the antibody to be used encompasses such human antibodies.

When an antibody is produced by isolating an antibody gene once, and introducing the gene into an appropriate host, an appropriate combination of a host and an expression vector can be used. When a eukaryotic cell is used as the host, an animal cell, a plant cell, or a fungal cell can be used. As the animal cell, (1) mammal cells such as CHO, COS, myeloma, BHK (baby hamster kidney), HeLa, and Vero, (2) amphibian cells such as Xenopus oocyte, and (3) insect cells such as sf9, sf21, and Tn5 are known. As the plant cell, a cell derived from the genus *Nicotiana,* such as a cell derived from *Nicotiana tabacum,* is known, and this cell may be callus cultured. As the fungal cell, yeast, for example, the genus *Saccharomyces,* such as *Saccharomyces serevisiae,* and filamentous fungus, for example, the genus *Aspergillus* such as *Aspergillus niger* are known. When a prokaryotic cell is used, there is a production system using a bacterial cell. As the bacterial cell, *Escherichia coli* (*E. coli*) and *Bacillus subtilis* are known. When a target antibody gene is introduced into such a cell by transformation, and the thus transformed cell is cultured *in vitro,* the antibody can be obtained.

Besides, the antibody to be used in the pharmaceutical formulation encompasses a modified antibody. For example, antibodies binding to various molecules of polyethylene glycol (PEG), cytotoxic drugs and the like can be used (Farmaco. 1999 Aug 30; 54(8): 497-516, Cancer J. 2008 May-June; 14(3): 154-69). Such a modified antibody can be obtained by chemically modifying an antibody. Such a method has been already established in this field.

In one aspect of the present invention, the antibody of the present disclosure may be a chimeric antibody. A chimeric antibody is described in, for example, US Patent No. 4,816,567, and Morrison et al., Proc. Natl. Acad. Sci. USA, 81: 6851-6855 (1984). A chimeric antibody may contain a non-human variable region (variable region derived from, for example, a non-human primate such as a monkey, or a mouse, a rat, a hamster, a rabbit or the like) and a human constant region.

In one aspect of the present invention, the antibody of the present disclosure may be a humanized antibody. Representatively, a humanized antibody is humanized for reducing immunogenicity in a human with specificity and affinity of a parent non-human antibody retained. A humanized antibody representatively contains one or more variable regions, and an HVR, for example, CDR derived from a non-human antibody (or a part thereof) and FR derived from a human antibody sequence (or a part thereof) are present therein. A humanized antibody can optionally contain at least a part of a human constant region. In one embodiment, amino acid residues of FR in a humanized antibody may be substituted with corresponding amino acid residues of a non-human antibody (for example, an antibody from which HVR residues are derived) for, for example, retaining or improving specificity and affinity of the antibody.

A humanized antibody and a method for producing the same are reviewed in, for example, the following (Almagro and Fransson, Front. Biosci. 13: 1619-1633 (2008)), and are described in, for example, the following: Riechmann et al., Nature 332: 323-329 (1988); Queen et al., Proc. Nat'l Acad. Sci. USA 86: 10029-10033 (1989); US Patent Nos. 5,821,337, 7,527,791, 6,982,321, and 7,087,409; Kashmiri et al., Methods 36: 25-34 (2005) (describing specificity determining region (SDR) grafting); Padlan, Mol. Immunol. 28: 489-498 (1991) (describing "resurfacing"); Dall'Acqua et al., Methods 36: 43-60 (2005) (describing "FR shuffling"); and Osbourn et al., Methods 36: 61-68 (2005) and Klimka et al., Br. J. Cancer, 83: 252-260 (2000) (describing "guided selection" approach to FR shuffling).

In one aspect of the present invention, a human framework that may be used in humanization may contain, for example, a framework selected by a "best fit" method (Sims et al., J. Immunol. 151: 2296 (1993)), a framework derived from a consensus sequence of a human antibody belonging to a specific subgroup of a heavy chain or light chain variable region (Carter et al., Proc. Natl. Acad. Sci. USA, 89: 4285 (1992), and Prest et al., J. Immunol., 151: 2623 (1993)), or a framework region derived from screening of FR library (Baca et al., J. Biol. Chem. 272: 10678-10684 (1997) and Rosok et al., J. Biol. Chem. 271: 22611-22618 (1996)).

In one aspect of the present invention, the antibody of the present disclosure may be a human antibody. A human antibody can be produced by various techniques. A human antibody is outlined in, for example, van Dijk and van de Winkel, Curr. Opin. Pharmacol. 5: 368-374 (2001) and Lonberg, Curr. Opin. Immunol. 20: 450-459 (2008). A human antibody may be prepared by administering an immunogen to a transgenic animal having been modified to produce a complete human antibody or a complete antibody containing a human variable region in response to an antigen. Such an animal representatively contains the entire or a part of human immunoglobulin locus, and the entire or a part of the human immunoglobulin locus is present in a state where it is substituted with an endogenous immunoglobulin locus, or it is randomly incorporated outside the chromosome or inside the chromosome of the animal. In such a transgenic mouse, endogenous immunoglobulin locus is usually inactivated. A method for obtaining a human antibody from a transgenic animal is reviewed in Lonberg, Nat. Biotech. 23: 1117-1125 (2005). Besides, make reference to, for example, US Patent Nos. 6,075,181 and 6,150,584 describing XENOMOUSE(TM) technology; US Patent No. 5,770,429 describing HUMAB(R) technology; US Patent No. 7,041,870 describing K-M MOUSE(R); and US2007/0061900 describing VELOCIMOUSE(R) technology. A human variable region from a complete antibody generated from such an animal may be further modified by, for example, combining with a different human constant region.

In another aspect of the present invention, a human antibody can be produced by a method based on a hybridoma. A human myeloma cell and a mouse-human heteromyeloma cell line for producing a human monoclonal antibody is described in the following (for example, Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991)). A human antibody generated through human B cell hybridoma technology is described in Li et al., Proc. Natl. Acad. Sci. USA, 103: 3557-3562 (2006). Other examples of the method include US Patent No. 7,189,826 (describing production of a monoclonal human IgM antibody from a hybridoma cell line), and Ni, Xiandai Mianyixue, 26 (4): 265-268 (2006) (describing a human-human hybridoma). Human hybridoma technology (trioma technology) is described in Vollmers and Brandlein, Histology and Histopathology, 20(3): 927-937 (2005), and Vollmers and Brandlein, Methods and Findings in Experimental and Clinical Pharmacology, 27(3): 185-91 (2005).

In another aspect of the present invention, a human antibody may also be generated by isolating an Fv clone variable domain sequence selected from human-derived phage display libraries. Such a variable region sequence can then be combined with a desired human constant region. Techniques for selecting a human antibody from antibody libraries will be described below.

In one aspect of the present invention, the antibody of the present disclosure may be isolated by screening a combinatorial library for an antibody having one or more desired activities. For example, a method for creating a phage display library, a method for screening such a library for an antibody having a desired binding characteristic, and the like are known in this technical field. Such methods are reviewed in Hoogenboom et al. in Methods in Molecular Biology 178:1-37 (O'Brien et al., ed., Human Press, Totowa, NJ, 2001), and are described in, for example, McCafferty et al., Nature 348: 552-554; Clackson et al., Nature 352: 624-628 (1991); Marks et al., J. Mol. Biol. 222: 581-597 (1992); Marks and Bradbury, Molecular Biology 248: 161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In a specific phage display method employed in one aspect of the present invention, repertoires of VH and VL can be separately cloned by polymerase chain reaction (PCR), and recombined randomly in phage libraries, and the phage libraries can then be screened for antigen-binding phage as described in Winter et al., Ann. Rev. Immunol., 12: 433-455 (1994). A phage displays an antibody fragment such as scFv or Fab. Libraries from immunized sources can provide high-affinity antibodies to the immunogen without the requirement of constructing hybridomas. In another embodiment, a naive repertoire can be cloned (for example, from a human) to provide a single source of antibodies to a wide range of non-self or self-antigens without any immunization as described by Griffiths et al., EMBO J, 12: 725-734 (1993). In a still another embodiment, naive libraries can also be created synthetically by cloning unrearranged V-gene segments from stem cells, and using PCR primers containing a random sequence encoding the highly variable region CDR3 and to accomplish rearrangement *in vitro,* as described in Hoogenboom and Winter, J. Mol. Biol., 227: 381-388 (1992). Examples of patent publications describing human antibody phage libraries include US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

An antibody or an antibody fragment isolated from a human antibody library is herein regarded as a human antibody or a human antibody fragment.

In one aspect of the present invention, the antibody of the present disclosure is a multispecific antibody (such as a bispecific antibody). A multispecific antibody is an antibody having binding specificities in at least two different sites (for example, a monoclonal antibody). In one embodiment, one of the binding specificities is specificity to an antigen, and the other is specificity to another antigen. In another embodiment, a bispecific antibody may bind to two epitopes of different antigens. The bispecific antibody may be used for localizing a cytotoxic agent in a cell expressing the antigen. The bispecific antibody may be prepared as a full-length antibody or an antibody fragment.

A method for producing a multispecific antibody is not limited, and examples include recombinant co-expression of two immunoglobulin heavy chain-light chain pairs having different specificities (for example, Milstein and Cuello, Nature 305: 537 (1983), WO93/08829, and Traunecker et al., EMBO J. 10: 3655 (1991)), and knob-in-hole technology (for example, US Patent No. 5,731,168). A multispecific antibody may be produced by controlling electrostatic steering effects for producing an Fc heterodimer molecule (for example, WO2009/089004 A1); cross-linking two or more antibodies or antibody fragments (for example, US Patent No. 4,676,980 and Brennan et al., Science, 229: 81 (1985)); producing an antibody having two specificities with leucine zipper (for example, Kostelny et al., J. Immunol., 148 (5): 1547-1553 (1992)); producing a bispecific antibody fragment by "diabody" technology (for example, Hollinger et al., Proc. Natl. Acad. Sci. USA, 90: 6444-6448 (1993)); using a scFv dimer (for example, Gruber et al., J. Immunol., 152: 5368 (1994)); or preparing a trispecific antibody (for example, Tutt et al., J. Immunol. 147: 60 (1991)). Alternatively, the multispecific antibody may be an antibody engineered to have three or more functional antigen-binding sites, including an "octopus antibody" (for example, US2006/0025576).

In one aspect of the present invention, the antibody or the antibody fragment thereof of the present disclosure may be a "dual acting Fab" or "DAF" containing one antigen-binding site binding to the antigen and another different antigen (for example, US2008/0069820).

In one aspect, a variant (mutant) of an amino acid sequence of the antibody of the present disclosure can be prepared by introducing an appropriate modification to a nucleic acid encoding a molecule of the antibody, or by synthesizing a peptide. Such a modification may be performed through one of or an appropriate combination of a plurality of deletion, insertion, and substitution of an arbitrary amino acid (residue) in the amino acid sequence. An arbitrary combination of deletion, insertion, and substitution can be employed as long as a final construct possesses a desired characteristic (for example, antigen-binding property).

In one aspect of the present invention, when an antigen variant (mutant) resulting from one of or a plurality of amino acid substitutions is provided, a target site for introducing substitution mutation can contain HVR and FR.

Examples of the antibody used in the pharmaceutical formulation include, but are not limited to, an anti-tissue factor antibody, anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, an anti-VLA4 antibody, an anti-HM1.24 antibody, an anti-parathyroid gland hormone-related peptide antibody (anti-PTHrP antibody), an anti-ganglioside GM3 antibody, an anti-TPO receptor agonist antibody, a coagulation factor VIII substitution antibody, an anti-IL31 receptor antibody, an anti-HLA antibody, an anti-AXI, antibody, an anti-CXCR4 antibody, an anti-NR10 antibody, and a bispecific antibody of factor IX and factor X.

Examples of a preferable reshaped humanized antibody to be used in the pharmaceutical formulation include a humanized anti-interleukin 6 (IL-6) receptor antibody (tocilizumab, hPM-1, or MRA, see WO92/19759), a humanized anti-HM1.24 antigen monoclonal antibody (see WO98/14580), a humanized anti-parathyroid gland hormone-related peptide antibody (anti-PTHrP antibody) (see WO98/13388), a humanized anti-tissue factor antibody (see WO99/51743), a humanized anti-glypican-3 IgG1**κ** antibody (codrituzumab, GC33, see WO2006/006693), a humanized anti-NR10 antibody (see WO2009/072604), and a bispecific humanized antibody of factor IX and factor X (ACE910, see WO2012/067176).

In one aspect of the present invention, the pharmaceutical formulation can be prepared, if necessary, by mixing with an appropriate pharmaceutically acceptable carrier, medium, or the like into a liquid formulation. The solvent of a liquid formulation is water or a pharmaceutically acceptable organic solvent. Examples of such an organic solvent include propylene glycol (1,2-propanediol), polyethylene glycol 300, polyethylene glycol 400, ethanol, glycerol, and acetic acid. Examples of appropriate pharmaceutically acceptable carrier and medium include sterilized water and physiological saline, a stabilizing agent, an antioxidant (such as ascorbic acid), a buffer (such as phosphoric acid, citric acid, histidine, or another organic acid), an antiseptic agent, a surfactant (such as PEG, or Tween), a chelating agent (such as EDTA), and a binding agent. Other low molecular weight polypeptides, proteins such as serum albumin, gelatin, and immunoglobulin, amino acids such as glycine, glutamine, asparagine, glutamic acid, aspartic acid, methionine, arginine, and lysine, sugars and carbohydrates such as polysaccharides and monosaccharides, and sugar alcohols such as mannitol and sorbitol may be contained. In the case of obtaining a solution for injection, examples include physiological saline, an isotonic solution containing glucose or another auxiliary drug, such as D-sorbitol, D-mannose, D-mannitol, or sodium chloride, and it may be used together with an appropriate solubilizing agent such as alcohol (such as ethanol), polyalcohol (such as propylene glycol, or PEG), and a nonionic surfactant (such as polysorbate 80, polysorbate 20, poloxamer 188, or HCO-50).

In one aspect of the present invention, the buffer to be used in a liquid formulation is prepared using a substance for retaining the pH of the solution. In one aspect of the present invention, a liquid formulation containing high concentration of an antibody has a pH of the solution of preferably 4.5 to 7.5, more preferably 5.0 to 7.0, and even more preferably 5.5 to 6.5. In one aspect of the present invention, a usable buffer can adjust the pH within this range, and is pharmaceutically acceptable. Such a buffer is known to those skilled in the art in the field of liquid formulation, and inorganic salts such as phosphates (sodium or potassium) and sodium bicarbonate; organic salts such as citrates (sodium or potassium), sodium acetate, and sodium succinate; and acids such as phosphoric acid, carbonic acid, citric acid, succinic acid, malic acid, and gluconic acid can be used. Alternatively, Tris buffers, and Good's buffers such as MES, MOPS, and HEPES, histidine (such as histidine hydrochloride), glycine, and such can be used.

Generally, the concentration of the buffer is 1 to 500 mmol/L, preferably 5 to 100 mmol/L and more preferably 10 to 20 mmol/L. In using a histidine buffer, the buffer preferably contains 5 to 25 mmol/L histidine, and more preferably contains 10 to 20 mmol/L histidine.

In one aspect of the present invention, a liquid formulation containing a high concentration of an antibody is preferably stabilized by addition of a stabilizing agent suitable for the antibody corresponding to the active ingredient. In one aspect of the present invention, no significant change is observed in a "stable" liquid formulation containing a high concentration of an antibody for at least 12 months, preferably for 2 years, and more preferably for 3 years at refrigeration temperature (2 to 8°C); or for at least 3 months, preferably for 6 months, and more preferably for 1 year at room temperature (22 to 28°C). For example, a total amount of dimers and decomposed matters after storage at 5°C for 2 years is 5.0% or less, preferably 2% or less, and more preferably 1.5% or less, or the total amount of dimers and decomposed matters after storage at 25°C for 6 months is 5.0% or less, preferably 2% or less, and more preferably 1.5% or less.

In one aspect of the present invention, representative examples of the surfactant include nonionic surfactants, for example, sorbitan fatty acid esters such as sorbitan monocaprylate, sorbitan monolaurate, and sorbitan monopalmitate; glycerin fatty acid esters such as glycerin monocaprylate, glycerin monomyristate, and glycerin monostearate; polyglycerin fatty acid esters such as decaglyceryl monostearate, decaglyceryl distearate, and decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sorbit fatty acid esters such as polyoxyethylene sorbit tetrastearate and polyoxyethylene sorbit tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether, and polyoxyethylene polyoxypropylene cetyl ether; polyoxyethylene alkyl phenyl ethers such as polyoxyethylene nonylphenyl ether; polyoxyethylene hydrogenated castor oils such as polyoxyethylene castor oil, and polyoxyethylene hydrogenated castor oil (polyoxyethylene hydrogen castor oil); polyoxyethylene beeswax derivatives such as polyoxyethylene sorbit beeswax; polyoxyethylene lanolin derivatives such as polyoxyethylene lanolin; polyoxyethylene fatty acid amides such as polyoxyethylene stearic acid amide that have HLB 6 to 18; anionic surfactants, for example, alkyl sulfates having an alkyl group having 10 to 18 carbon atoms such as sodium cetyl sulfate, sodium lauryl sulfate, and sodium oleyl sulfate; polyoxyethylene alkyl ether sulfates such as sodium polyoxyethylene lauryl sulfate having an average added molar number of ethylene oxide of 2 to 4 and an alkyl group having 10 to 18 carbon atoms; alkylsulfosuccinic acid esters having an alkyl group having 8 to 18 carbon atoms such as sodium lauryl sulfosuccinate ester; and natural surfactants, for example, lecithin and glycerophospholipids; sphingo-phospholipids such as sphingomyelin; and sucrose fatty acid esters of fatty acids having 12 to 18 carbon atoms. In one aspect of the present invention, one or more of these surfactants can be added in combination to the formulation.

Preferred surfactants include polyoxyethylene sorbitan fatty acid esters and polyoxyethylene polyoxypropylene alkyl ethers, polysorbates 20, 21, 40, 60, 65, 80, 81, 85, and Pluronic(R) surfactants are particularly preferred, and polysorbates 20 and 80 and Pluronic(R) F-68 (poloxamer 188) are most preferred.

In one aspect of the present invention, an amount of the surfactant to be added to the antibody formulation is generally 0.0001 to 10% (mg/mL), preferably 0.001 to 5%, and more preferably 0.005 to 3%.

To the formulation of the present invention, a cryoprotectant, a suspending agent, a dissolution assisting agent, a tonicity agent, a preservative, an adsorption inhibitor, a diluent, an excipient, a pH adjustor, a soothing agent, a sulfur-containing reducing agent, an antioxidant and the like can be appropriately added.

Examples of the cryoprotectant include sugars such as trehalose, sucrose, and sorbitol.

Examples of the dissolution assisting agent include polyoxyethylene hydrogenated castor oil, polysorbate 80, nicotinamide, polyoxyethylene sorbitan monolaurate, macrogol, and castor oil fatty acid ethyl ester.

Examples of the tonicity agent include sodium chloride, potassium chloride, and calcium chloride.

Examples of the preservative include methyl parahydroxybenzoate, ethyl parahydroxybenzoate, sorbic acid, phenol, cresol, and chlorocresol.

Examples of the adsorption inhibitor include human serum albumin, lecithin, dextran, an ethylene oxide/propylene oxide copolymer, hydroxypropyl cellulose, methyl cellulose, polyoxyethylene hydrogenated castor oil, and polyethylene glycol.

Examples of the sulfur-containing reducing agent include those containing sulfhydryl groups such as N-acetylcysteine, N-acetylhomocysteine, thioctic acid, thiodiglycol, thioethanol amine, thioglycerol, thiosorbitol, thioglycolic acid and salts thereof, sodium thiosulfate, glutathione, and thioalkanoic acids having 1 to 7 carbon atoms.

Examples of the antioxidant include erythorbic acid, dibutylhydroxytoluene, butylhydroxyanisole, α-tocopherol, tocopherol acetate, L-ascorbic acid and salts thereof, L-ascorbic acid palmitate, L-ascorbic acid stearate, sodium hydrogen sulfite, sodium sulfite, triamyl gallate, propyl gallate, and chelating agents such as disodium ethylenediamine tetraacetate (EDTA), sodium pyrophosphate, and sodium metaphosphate.

In one aspect of the present invention, the pharmaceutical formulation is used for treating an autoimmune disease, an immune disease, an infectious disease, an inflammatory disease, a nervous system disease, and a tumor disease and a neoplasm disease including cancer. In a specific embodiment, the pharmaceutical is used for treating congestive heart failure (CHF), ischemia-induced severe arrhythmia, hypercholesteremia, vasculitis, rosacea, acne, eczema, myocarditis, and other states of cardiac muscle, Kawasaki Disease, systemic lupus erythematosus, diabetes, spondylosis, synovial fibroblast, and bone marrow stroma; bone loss; Paget's disease, giant cell tumor of bone; breast cancer; disuse bone loss; malnutrition, periodontal disease, Gaucher's disease, Langerhans cell histiocytosis, spinal cord injury, acute purulent arthritis, osteomalacia, Cushing's syndrome, monostotic fibrous dysplasia, polyostotic fibrous dysplasia, periodontal membrane reconstruction, and fracture; sarcoidosis; melanoma, prostate cancer, pancreas cancer, osteolytic bone cancer, breast cancer, lung cancer, stomach cancer, renal cancer, and rectal cancer; bone metastasis, bone pain management, humoral hypercalcemia of malignancy, ankylosing spondylitis, and other spondylarthrosis; transplant rejection, viral infection, hematological neoplasm, and neoplasm-like state such as Hodgkin's lymphoma; non-Hodgkin's lymphoma (Burkitt's lymphoma, small lymphocytic lymphoma/chronic lymphatic leukemia, mycosis fungoides, mantle cell lymphoma, follicularlymphoma, diffuse large B-cell lymphoma, marginal zone lymphoma, hairy cell leukemia, and lymphoplasmacytic leukemia), tumors of lymphocyte progenitor cells including B-cell acute lymphoblastic leukemia/lymphoma and T-cell acute lymphoblastic leukemia/lymphoma, thymoma, peripheral T-cell leukemia, adult T-cell leukemia/T-cell lymphoma, and tumors of mature T-cell and nature NK cell including large granular lymphocyte leukemia, Langerhans cell histiocytosis, AML with maturation, AML without differentiation, acute myelocytic leukemias including acute promyelocytic leukemia, acute myelomonocytic leukemia, and acute monocytic leukemia, myelodysplastic syndromes, and bone marrow neoplasms such as chronic myeloproliferative disease including chronic myelocytic leukemia, tumors of central nervous system, for example, brain tumor (glioma, neuroblastoma, astrocytoma, medulloblastoma, ependymoma, and retinoblastoma), solid tumor (nasopharyngeal cancer, basal cell carcinoma, pancreatic cancer, cholangiocarcinoma, Kaposi's sarcoma, testicular cancer, uterine cancer, vaginal cancer or cervical cancer, ovarian cancer, primary hepatic cancer, or endometrial cancer, and tumors of vascular system (angiosarcoma and hemangiopericytoma), osteoporosis, hepatitis, HIV, AIDS, spondyloarthritis, rheumatoid arthritis, inflammatory bowel disease (IBD), sepsis and septic shock, Crohn's disease, psoriasis, scleroderma, graft versus host disease (GVHD), allogeneic islet graft rejection, multiple myeloma (MM), myelodysplastic syndromes (MDS), and hematologic malignancy such as acute myelogenous leukemia (AML), inflammation related to tumor, peripheral nerve injury, or demyelinating disease. In a specific embodiment, the pharmaceutical is used for treating psoriasis vulgaris, pancreatitis, ulcerative colitis, non-Hodgkin's lymphoma, breast cancer, colorectal cancer, mesothelioma, soft tissue sarcoma, juvenile idiopathic arthritis, macular degeneration, respiratory syncytial virus, Crohn's disease, rheumatoid arthritis, psoriatic arthritis, Castleman's disease, ankylosing spondylitis, osteoporosis, treatment-induced bone loss, bone metastasis, multiple myeloma, Alzheimer's disease, glaucoma, Sjogren's disease, Still's disease, multiple sclerosis, hyperimmunoglobulinemia, anemia, mesangial proliferative nephritis, and asthma.

In one aspect of the present invention, an antigen to which the antibody has a specific binding property can be a transmembrane molecule (such as a receptor) or a ligand such as a growth factor. Representative examples of the antigen include a molecule such as renin; growth hormones including human growth hormone and bovine growth hormone; a growth hormone-releasing factor; parathyroid gland hormone; thyroid stimulating hormone; lipoprotein; α-1 anti-trypsin; insulin A chain; insulin B chain; proinsulin; follicle-stimulating hormone; calcitonin; luteinizing hormone; glucagon; coagulation factors such as factor VIIIC, factor IX, tissue factor (TF), and von Willebrand factor; anticoagulation factors such as protein C; atrial natriuretic factor; a pulmonary surfactant; plasminogen activators such as urokinase, and human urinary or tissue plasminogen activator (t-PA); bombesin; thrombin; hematopoietic cell growth factor; tumor necrosis factor-α and -β; enkephalinase; RANTES (regulated on activation normally T-cell expressed and secreted); human macrophage inflammatory protein (MIP-1-α); serum albumin such as human serum albumin; Mullerian-inhibiting substance; relaxin A chain; relaxin B chain; prorelaxin; mouse gonadotropin releasing hormone-related peptide; microbial proteins such as β-lactamase; DNAse; IgE; cytotoxic T-lymphocyte related antigens (CTLA) such as CTLA-4; inhibin; activin; vascular endothelial growth factor (VEGF); hormone or growth factor receptors; protein A or D; rheumatoid factor; neurotrophic factor, such as bone-derived neurotrophic factor (BDNF), or neurotrophin-3, -4, -5, or -6 (NT-3, NT-4, NT-5, or NT-6), and nerve growth factor such as NGF-b; platelet-derived growth factor (PDGF); fibroblast growth factors such as aFGF and bFGF; epidermal growth factor (EGF); transforming growth factors (TGF) such as TGF-α, and TGF-β including TGF-b1, TGF-b2, TGF-b3, TGF-b4, and TGF-b5; tumor necrosis factors (TNF) such as TNF-α and TNF-β; insulin-like growth factor-I and -II (IGF-I and IGF-II); des(1-3)-1GF-I (brain IGF-I), and insulin-like growth factor binding protein; CD proteins such as CD3, CD4, CD8, CD19, CD20, CD22, and CD40; erythropoietin; bone morphogenetic protein; an antitoxin; bone morphogenetic protein (BMP); interferons such as interferon-α, -β, and -γ; colony stimulating factors (CSF) such as M-CSF, GM-CSF, and G-CSF; interleukins (IL) such as IL-1, IL-2, IL-3, IL-4, IL-5, IL-6, IL-7, IL-8, IL-9, and IL-10; superoxide dismutase; a T-cell receptor; a surface membrane protein; a decay acceleration factor; viral antigens such as a part of AIDS envelope; a transport protein; a homing receptor; addressin; regulatory proteins; integrins such as CD11a, CD11b, CD11c, CD18, ICAM, VLA-4, and VCAM; tumor-related antigens such as HER2, HER3, and HER4 receptors; and a fragment of any of the above-described polypeptides.

In one aspect of the present invention, examples of a molecular target of the antibody comprised therein include CD proteins such as CD3, CD4, CD8, CD19, CD20, CD22, CD34, and CD40; members of the ErbB receptor family such as EGF receptor, and HER2, HER3, or HER4 receptor; B-cell surface antigens such as CD20 and BR3; members of the tumor necrosis receptor superfamily including DR5; prostate stem cell antigen (PSCA); cell adhesion molecules such as LFA-1, Mac1, p150.95, VLA-4, ICAM-1, VCAM, and αv/β3 integrins including any one of α4/β7 integrin, and α or β subunit thereof (such as an anti-CD11a, anti-CD18, or anti-CD11b antibody); growth factors such as VEGF and receptors thereof; tissue factors (TF); tumor necrosis factors (TNF), such as TNF-α or TNF-β, and α-interferon (α-IFN); interleukins such as IL-8; IgE; blood group antigens; flk2/flk3 receptor; obesity (OB) receptor; mp1 receptor; CTLA-4; and protein C.

### (4) Container

In one aspect of the present invention, examples of a container to be filled with the pharmaceutical formulation include a syringe and a cartridge.

In one aspect of the present invention, the term "pre-filled syringe" means a syringe used as a container and filled with a liquid composition. In one embodiment, a pre-filled syringe contains a pharmaceutical composition for administration to a patient filled in the syringe. Here, the syringe may be covered with a syringe closure, such as, but not limited to, a stopper. In one embodiment, the syringe was filled with the composition in a filling facility for production. In one embodiment, the syringe is sterilized before being filled with the composition therein. In one embodiment, the pre-filled syringe may be stored, before administering the composition to a patient, for a period of one day, or at least 7 days, or at least 14 days, or at least 1 month, or at least 6 months, or at least 1 year, or at least 2 years. In one embodiment, the pre-filled syringe is exposed to storage and/or transportation conditions.

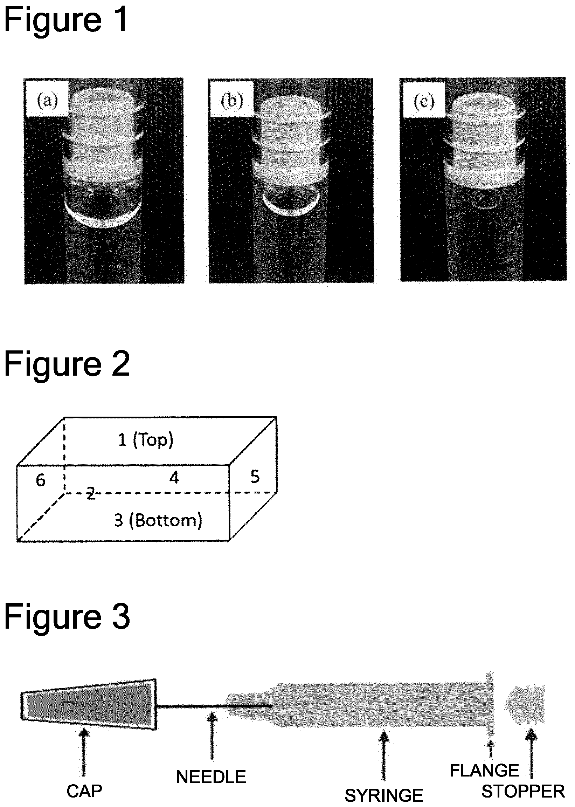

In one embodiment of the present invention, the pre-filled syringe is exposed to mechanical stress. Examples of the mechanical stress include, but are not limited to, drop stress, vibration stress, and rotation stress. In one embodiment of the present invention, the pre-filled syringe is exposed to drop stress once, twice, three times, four times, five times, six times, seven times, eight times, nine times, ten times, eleven times, twelve times, thirteen times, fourteen times, fifteen times, sixteen times, seventeen times, eighteen times, nineteen times, twelve times, twenty one times, twenty two times, twenty three times, twenty four times, twenty five times, twenty five times or more, thirty times or more, or forty times or more. Stress applied to the pre-filled syringe at the time of drop is varied not only depending on the number of dropping times but also a drop height, a drop direction and the like. The drop height is, but not limited to, for example, 38.1 cm described in American Society for Testing and Materials (ASTM) D4169. Besides, for purposes of applying equivalent drop stress with high reproducibility, the pre-filled syringe may be appropriately packaged so as not to change the direction of the pre-filled syringe during the drop. An example of the packaging includes, but is not limited to, "putting it in a tray, and stacking trays", and "packaging stacked trays in a cardboard box having surfaces numbered as illustrated in Figure 2 with the needle of the pre-filled syringe facing the surface 2". Preferably, with respect to the drop height and direction, the pre-filled syringe is dropped from the height of 38.1 cm with the surface facing downward in the drop changed in the order of the surface 1, the surface 2, the surface 3 and the surface 4. With this drop defined as one set of the drop, two sets of the drop are performed for applying the drop stress once.

As shown in Figure 3, the pre-filled syringe includes a syringe for administering a drug. Also, the pre-filled syringe includes a stopper to be inserted into the syringe. The pre-filled syringe further includes a needle to be connected to the syringe. The pre-filled syringe includes a cap for capping the needle. The drug is a liquid drug, and is, for example, a protein formulation.

The syringe includes a barrel formed in a cylindrical shape having a leading end portion and a base end portion. Also, the syringe is substantially cylindrical.

The barrel is a member for containing a drug therein. The barrel of the present embodiment includes, in addition to the leading end portion and the base end portion, a cylindrical portion connecting the leading end portion and the base end portion (see Figure 3). Also, the barrel has a flange portion extending outward (radially outward direction of the cylindrical portion) from the entire outer circumference of the other end in the axial direction of the cylindrical portion.

The barrel is formed, for example, from a material that is transparent and can withstand an internal pressure applied in administering the drug. Specifically, the material of the barrel is a resin containing cyclic olefin such as norbornene in a repeating unit. More specifically, examples of the material of the barrel include transparent resins such as COP (cycloolefin polymer) that is a homopolymer of cyclic olefin, and COC (cycloolefin copolymer) that is a copolymer of cyclic olefin and ethylene or the like. The barrel may be made of PP (polypropylene) or glass.

On the inner surface of the barrel (for example, the inner surface of the cylindrical portion), silicone oil may be applied as a lubricant for reducing sliding resistance of a piston against the inner surface of the barrel.

In one aspect of the present invention, the silicone oil is polydimethylsiloxane. Some non-restrictive examples of the polydimethylsiloxane include Dow Corning(R) 360 Medical Fluid, non-restrictively including Dow Corning (R) 360 Medical Fluid having a viscosity of 350 centistokes, Dow Corning (R) 360 Medical Fluid having a viscosity of 1,000 centistokes, Dow Corning (R) 360 Medical Fluid having a viscosity of 12,500 centistokes, and Dow Corning (R) MDX4-4159 fluid.

In one aspect of the present invention, the size (standard) of a volume of the syringe is not particularly limited. Specifically, in one aspect of the present invention, the advantageous effect is notable in the case of a small-sized syringe having a volume of 0.5 mL to 5.0 mL, and preferably 1 mL. The volume of a solution contained in a syringe of 1 mL standard is within a range of 0.1 to 1.2 mL, and preferably within a range of 0.2 to 1.1 mL. The volume of the solution contained in a syringe of 2.5 mL standard is within a range of 0.1 to 2.5 mL, and preferably within a range of 0.3 to 2.3 mL. When the pharmaceutical formulation contains an aqueous solution, the volume of the aqueous solution contained in a syringe of 1 mL standard is within a range of 0.1 to 1.2 mL, and preferably within a range of 0.2 to 1.1 mL. The volume of the aqueous solution contained in a syringe of 2.5 mL standard is within a range of 0.1 to 2.5 mL, and preferably within a range of 0.3 to 2.3 mL.

In one aspect of the present invention, the size (standard) of a volume of the cartridge is not particularly limited. Specifically, the volume may be, but is not limited to, 0.5 mL to 20.0 mL, for example, 1.0 mL, 1.5 mL, 1.8 mL, 2.0 mL, 2.2 mL, 3.0 mL, 5.0 mL, 10.0 mL, 15.0 mL, or 20.0 mL.

In one aspect of the present invention, the cartridge to be used may be a standard cartridge for injection made of plastic or glass. The dimension and tolerance of a glass injection cartridge are defined in International Standard ISO 13926-1. Stoppers and seals (caps and discs) are described in International Standard ISO 13926-2 and 3. The dimension and tolerance of a ready-to-fill syringe or pre-filled syringe are defined in International Standard ISO 11040-4. In one embodiment, the cartridge to be used may be a cartridge for injection made of plastic or glass that meets one or more of the above-discussed International Standards. In another aspect of the present invention, the cartridge to be used may be a cartridge for injection made of plastic or glass that does not comply with international standards such as ISO.

### (5) System

In one aspect of the present invention, a system for determining, in a pharmaceutical formulation comprising a protein as an active ingredient in a solution, a protein having a high risk of forming particles in a solution is provided. This system includes means for constructing a three-dimensional structure model of a protein based on an amino acid sequence of the protein by homology modeling or antibody modeling; means for specifying, in a surface of the obtained model, a portion where hydrophobic residues are accumulated in a cluster, and a portion where residues with a charge are accumulated in a cluster as a hydrophobic patch and a charged patch, respectively, and calculating areas of the patches; means for calculating a sum of areas of top 5 hydrophobic patches ranked according to the area (X ((angstrom)²)), and a total area of charged patches (Y ((angstrom)²)); and means for determining that a protein having a "X + Y × 1.5" value of 1,700 or greater is a protein having a high risk of forming particles in a solution.

In one aspect of the present invention, the system is a system for performing a method for determining a protein having a high risk of forming particles in a solution, and can perform the method by installing the following program in a determination apparatus, such as a computer.

In one aspect of the present invention, the program is a program for causing a computer to operate the respective means in the system, and even when a determination apparatus, in a pharmaceutical formulation comprising a protein as an active ingredient in a solution, a protein having a high risk of forming particles in a solution, is a general-purpose apparatus, the program is a computer program that can make the general-purpose apparatus usable as the determination apparatus when installed in the general-purpose apparatus. In one aspect of the present invention, the computer program is not always necessary to be installed in the determination apparatus but can be stored in, for example, a recording medium to be provided. Here, the term "recording medium" refers to a medium capable of carrying a program that does not occupy a space by itself, and examples include a flexible disk, a hard disk, a CD-R, a CD-RW, an MO (magneto-optical disk), a DVD-R, a DVD-RW, and a flash memory. Besides, the computer program can be transmitted from a computer storing the computer program to another computer or apparatus via a communication line. In one aspect of the present invention, the computer program encompasses such a computer program stored in a computer, and a computer program under transmission.

### (Program for apparatus determining protein having high risk of forming particles in solution based on hydrophobic patch and charged patch)

In one aspect of the present invention, the present invention relates to a program that is used in an apparatus for determining, in a pharmaceutical formulation comprising a protein as an active ingredient in a solution, a protein having a high risk of forming particles in a solution based on a hydrophobic patch and a charged patch, or is stored in a recording medium to be used.

The program causes the apparatus or a computer to execute means for constructing a three-dimensional structure model of a protein based on an amino acid sequence of the protein by homology modeling or antibody modeling; means for specifying, in a surface of the obtained model, a portion where hydrophobic residues are accumulated in a cluster, and a portion where residues with a charge are accumulated in a cluster as a hydrophobic patch and a charged patch, respectively, and calculating areas of the patches; means for calculating a sum of areas of top 5 hydrophobic patches ranked according to the area (X ((angstrom)²)), and a total area of charged patches (Y ((angstrom)²)); and means for determining that a protein having a "X + Y × 1.5" value of 1,700 or greater is a protein having a high risk of forming particles in a solution.

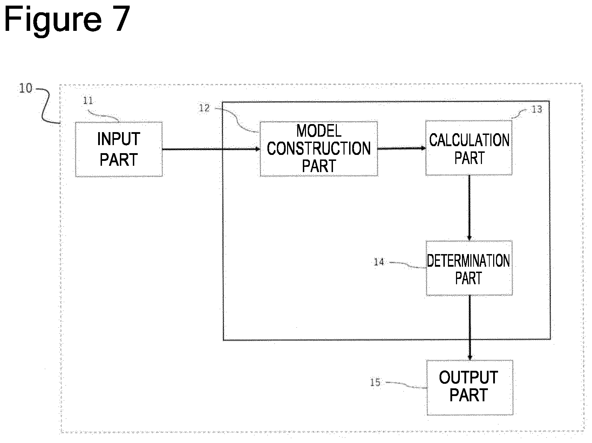

In a configuration diagram of an apparatus (10) for determining a protein having a high risk of forming particles in a solution illustrated in Figure 7, the means for constructing a model is executed by a model construction part (12) based on an amino acid sequence input through an input part (11). The means for specifying a hydrophobic patch and a charged patch, calculating areas of the patches, and calculating a sum of areas of top 5 hydrophobic patches ranked according to the area (X ((angstrom)²), and a total area of charged patches (Y ((angstrom)²) is performed by a calculation part (13). The means for determining that a protein having a "X + Y × 1.5" value of 1,700 or greater is a protein having a high risk of forming particles in a solution is performed by a determination part (14). A result of the determination is output from an output part (15). These means are executed by, for example, a CPU reading the computer program stored in an HDD.

The computer program cannot singly occupy a space, but can be stored in an information recording medium to be distributed. Here, the term "information recording medium" refers to, for example, a flexible disk, a hard disk, a CD-ROM, a CD-R, a CD-RW, an MO (magneto-optical disk), an MD, a DVD-R, a DVD-RW, a flash memory, or an IC card. The information recording medium is connected to a data input/output part of the apparatus, and thus, the computer program can be installed in a memory such as an HDD in the apparatus. Besides, the computer program can be transmitted from another computer storing the computer program via a communication line to the apparatus to be installed in a memory such as an HDD therein.

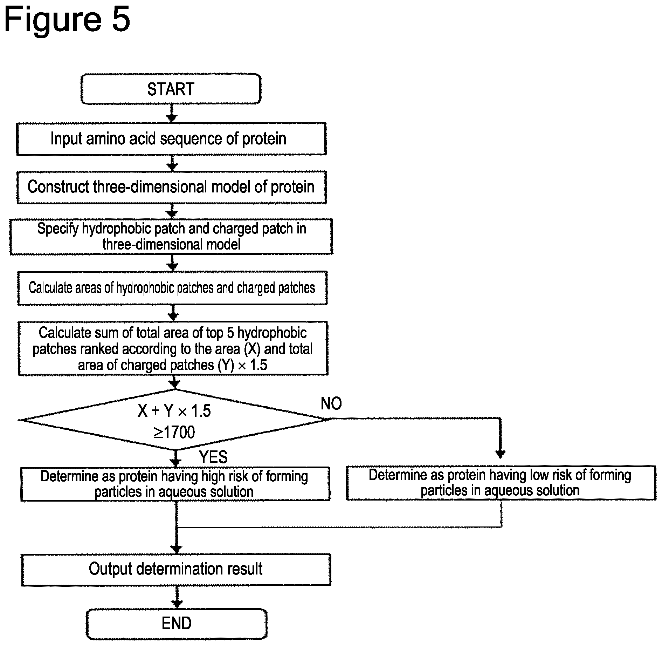

Figure 5 illustrates a flow of a process performed by the apparatus when the computer program for an apparatus for determining a protein having a high risk of forming particles in a solution is executed. The CPU of the apparatus reads the computer program for the apparatus stored in the memory such as the HDD or the like of the apparatus, and constructs a three-dimensional model of a protein based on an amino acid sequence of the protein by homology modeling or antibody modeling. Next, the CPU reads the computer program, and specifies, in the constructed model, a portion where hydrophobic residues are accumulated in a cluster, and a portion where residues with a charge are accumulated in a cluster as a hydrophobic patch and a charged patch, respectively, and calculates areas of the patches, and calculates a sum of areas of top 5 hydrophobic patches ranked according to the area (X ((angstrom)²)), and a total area of charged patches (Y ((angstrom)²)). Next, the CPU reads the computer program, and determines that a protein having a "X + Y × 1.5" value of 1,700 or greater is a protein having a high risk of forming particles in a solution.

### (Program for apparatus determining protein having high risk of forming particles in solution based on charged patch)

In one aspect of the present invention, the present invention relates to a program that is used in an apparatus for determining, in a pharmaceutical formulation comprising a protein as an active ingredient in a solution, a protein having a high risk of forming particles in a solution based on a charged patch, or is stored in a recording medium to be used.

The program causes the apparatus or computer to execute means for constructing a three-dimensional structure model of a protein based on an amino acid sequence of the protein by homology modeling or antibody modeling; means for specifying, in a surface of the obtained model, a portion where residues with a charge are accumulated in a cluster as a charged patch, and calculating a total area of charged patches (Y ((angstrom)²)); and means for determining that a protein having a Y value of 600 or greater is a protein having a high risk of forming particles in a solution.

The means for constructing a model is executed by a model construction part. The means for specifying a charged patch and calculating a total area of charged patches (Y ((angstrom)²)) is performed by a calculation part. The means for determining that a protein having a Y value of 600 or greater is a protein having a high risk of forming particles in a solution is performed by a determination part. These means are executed by, for example, a CPU reading a computer program stored in an HDD.

The computer program cannot singly occupy a space, but can be stored in an information recording medium to be distributed. Here, the term "information recording medium" refers to, for example, a flexible disk, a hard disk, a CD-ROM, a CD-R, a CD-RW, an MO (magneto-optical disk), an MD, a DVD-R, a DVD-RW, a flash memory, or an IC card. The information recording medium is connected to a data input/output part of the apparatus for determining a portion having a high risk of forming particles in a solution, and thus, the computer program can be installed in a memory such as an HDD in the apparatus. Besides, the computer program can be transmitted from another computer storing the computer program via a communication line to the apparatus to be installed in a memory such as an HDD therein.

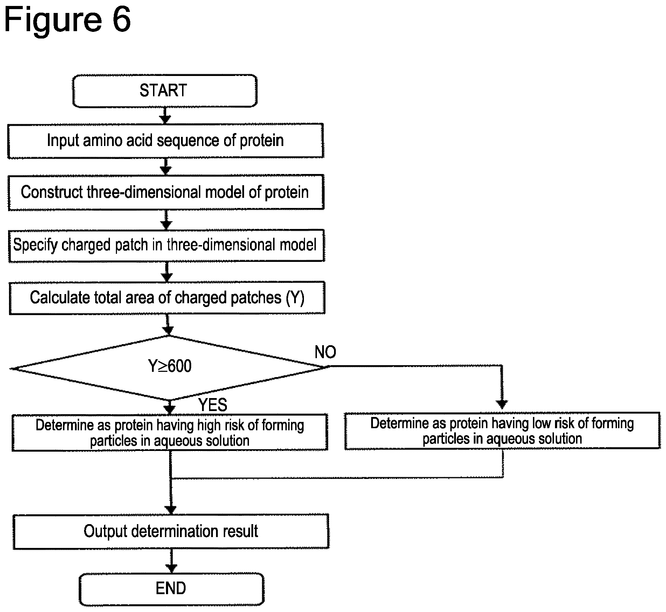

Figure 6 illustrates a flow of a process performed by the apparatus when the computer program for an apparatus for determining a protein having a high risk of forming particles in a solution is executed. The CPU of the apparatus reads the computer program for the apparatus stored in the memory such as the HDD or the like of the apparatus, and constructs a three-dimensional model of a protein based on an amino acid sequence of the protein by homology modeling or antibody modeling. Next, the CPU reads the computer program to specify, in the constructed model, a portion where residues with a charge are accumulated in a cluster as a charged patch, and calculates a total area of charged patches (Y ((angstrom)²)). Next, the CPU determines that a protein having a Y value of 600 or greater is a protein having a high risk of forming particles in a solution.

### EXAMPLES

### Example 1 Counting visually detectable particles

With respect to each of six antibodies of mAb1 (H-chain/SEQ ID NO: 1, L-chain/SEQ ID NO: 2; tocilizumab), mAb2 (H-chain/SEQ ID NOs: 3 and 4: common L-chain: SEQ ID NO: 5), mAb3 (H-chain/SEQ ID NO: 6, L-chain/SEQ ID NO: 7), mAb4 (humanized bispecific antibody having blood coagulation factor VIII (FVIII) cofactor function alternative activity), mAb5 (anti-latent myostatin sweeping humanized antibody), and mAb6 (combination of H-chain/SEQ ID NO: 8 and L-chain/SEQ ID NO: 9, and combination of H-chain/SEQ ID NO: 11 and L-chain/SEQ ID NO: 10; anti-HLA-DQ2.5 humanized bispecific antibody), an antibody-containing solution (each of mAb1 to mAb6: 50 mg/mL, buffer: 20 mmol/L histidine, stabilizer: 150 mmol/L arginine and 162 mmol/L aspartic acid, surfactant: 0.01 mg/mL poloxamer 188, pH 6.0) was prepared, and the resultant was filtrated with a 0.22 µm filter, then, 1.0 mL of the resultant solution was filled in a COP syringe with a 27G needle (1 mL standard) having been sterilized with radiation (25 kGy), and the resultant syringe was stoppered with a stopper. With respect to each of the filled and stoppered samples, Evaluation 1 of visually detectable particles was performed immediately after stoppering, and a sample determined to contain visually detectable particles in the syringe was excluded. Ten samples of each antibody were tested, andmAb6 were highly unstable as compared with other antibodies, and visually detectable particles were very highly frequently formed therein immediately after filling, and therefore, three syringes each were tested. Based on a standard curve created by the following method for measuring and setting an air volume, the position of the stopper of each syringe was adjusted so as to prepare samples having the air volume of 120 µL and 10 µL. For each sample of the air volume, Evaluation 2 of visually detectable particles was performed after storage at 5°C for 1 day.

### [Evaluation 1 of visually detectable particles]

The outer surface of the syringe container of each sample was cleaned, the syringe was slowly rotated or inverted at a position directly below a white light source, at a brightness of about 10,000 lx, and in front of a black background, and visual inspection was performed for about 30 seconds with naked eyes to examine whether or not visually detectable particles were present in the solution filled in the syringe.

### [Method for measuring/setting air volume]

The COP syringe with a 27G needle (1 mL standard) of each antibody solution-containing syringe sample filled with 1.0 mL of the antibody-containing solution and stoppered with a stopper was held with the needle facing upward, air was pushed out from the needle tip by ascending the air up to the base of the needle, and the position of the stopper was adjusted so that a distance from the flange to the stopper be 11 mm. The solution and the whole air contained in the syringe were injected into a tube with an inner diameter of 0.5 mm. The air volume in the syringe was calculated by measuring the length of an air space thus formed in the tube. This measurement was repeated 4 times, and the results revealed that an average air volume was 120 µL when the distance from the flange to the stopper was 11 mm. Next, 3 samples were prepared by letting air out from the needle tip of the syringe as much as possible, and the distance from the flange to the stopper was measured. The average distance from the flange to the stopper was 15.1 mm. Besides, the actual air volume was 3 µL. A standard curve of the distance from the flange to the stopper, and the air volume was created based on these measurement results, and the air volume was set based on the distance from the flange to the stopper. The conditions for the air volume thus set are shown in Table 1 below.

**[Table 1]**

| Table 1 Setting Conditions for Air Volume | |
|---|---|
| Distance from Flange to Stopper | Air Volume |
| 11 mm | 120 µL |
| 14.75 mm | 10 µL |

### [Evaluation 2 of visually detectable particles]

The outer surface of the syringe container of the sample was cleaned, the syringe was slowly rotated or inverted at a position directly below a white light source, at a brightness of about 8,000 lx, and in front of a black background, and visual inspection was performed for about 30 seconds to examine whether or not visually detectable particles were present in the solution filled in the syringe. Regarding a sample in which visually detectable particles were found to be present, the number of visually detectable particles in the syringe was counted with naked eyes by slowly rotating or inverting the syringe at a position directly below a white light source, at a brightness of about 8,000 lx, and in front of a black background.

### [Evaluation results]

The results of Evaluation 2 of visually detectable particles in the syringes after storing the samples at 5°C for 1 day are shown in Table 2 below.

**[Table 2]**

| Table 2 Evaluation Results of Visually Detectable Particles after Storage at 5°C for 1 Day | | | | | | |
|---|---|---|---|---|---|---|
| Sample Name | Filled Amount (mL) | Air Volume (µL) | Number of Visual Inspection Samples | Number of Samples Containing Visually Detectable Particles | Total Number of Visually Detectable Particles | Average of Visually Detectable Particles per Syringe |
| mAb1-120 | 1 | 120 | 10 | 0 | 0 | 0 |
| mAb1-10 | | 10 | 10 | 0 | 0 | 0 |
| mAb2-120 | 1 | 120 | 10 | 9 | 15 | 1.5 |
| mAb2-10 | | 10 | 10 | 6 | 10 | 1.0 |
| mAb3-120 | 1 | 120 | 10 | 7 | 13 | 1.3 |
| mAb3-10 | | 10 | 10 | 3 | 3 | 0.3 |
| mAb4-120 | 1 | 120 | 10 | 9 | 22 | 2.2 |
| mAb4-10 | | 10 | 10 | 4 | 5 | 0.5 |
| mAb5-120 | 1 | 120 | 10 | 4 | 6 | 0.6 |
| mAb5-10 | | 10 | 10 | 2 | 2 | 0.2 |
| mAb6-120 | 1 | 120 | 3 | 3 | 15 | 5.0 |
| mAb6-10 | | 10 | 3 | 3 | 6 | 2.0 |

### Example 2 Determination of conditions of high risk of forming particles

### 1. Construction of three-dimensional structure model of antibody

In the Antibody modeling function of Molecular Operating Environment (MOE), 2019.01 (Chemical Computing Group ULC), amino acid sequences of the six antibodies (mAb1 to mAb6) were input to be matched with individual templates for each of complementary determining regions (CDR), and the resultants were combined to construct a three-dimensional structure model of each antibody in a range of full length IgG. As conditions for calculation, Chains: VL and VH option was employed for a monospecific antibody, Chains: bispecific option was employed for some of bispecific antibodies, and Ig Immunoglobulin was employed for Model type. With respect to all the antibodies, crystal structure information evaluated as optimal by Antibody modeling function was used for template structures of a framework region and a complementary determining region. As a threshold of a gradient value (gradient limit) of energy minimization convergence of the structure, 0.1 kcal/mol/(angstrom)² was applied. Default values were used as other parameters. As for the force field, Amber 10: EHT, which is the standard of MOE 2019.01 was used.

Residues that were not contained in the antibody sequences of mAb2 and mAb3 but automatically complemented by MOE were deleted, and energy minimization was performed on residues around the deleted ones. Residues were not particularly deleted for mAb1.

### 2. Calculation of physical properties of three-dimensional structure models of antibodies

The three-dimensional structure models of the six antibodies (mAb1 to mAb6) constructed in 1. were input to comprehensively calculate a feature amount of each antibody by Protein properties function of MOE 2019.01. Except that Target pH was changed to 6, default values were used as the other parameters. Each feature amount thus output was stored as an MDB file.

With respect to each antibody, four feature amounts out of the calculated feature amounts, specifically, a sum of areas of top 5 hydrophobic patches among all hydrophobic patches ranked according to the area (Patch _hyd_5), a sum of areas of all the hydrophobic patches (Patch_hyd), a sum of areas of top 5 charged patches among all the charged patches ranked according to the area (Patch _ion_5), and a sum of areas of all the charged patches (Patch_ion) were extracted from the MDB file to examine correlation with experimental values. At this point, correlation with experimental values was also examined for a combination of two feature amounts. In the examination of the correlation, Pearson's product-moment correlation coefficient and Spearman's rank correlation coefficient using each feature amount as a variable, and using an average number of visually detectable particles per syringe of a sample having the air volume of 120 µL shown in Table 2 of Example 1 as a variable were used. All the feature amounts use a unit of the area of (angstrom)².

In all these calculations using MOE above, as for the molecular force field, Amber 10: EHT that is the standard of MOE 2019.01 was used.

The values of Patch_hyd_5, Patch_hyd, Patch_ion_5, and Patch_ion of the six antibodies (mAb1 to mAb6), and the combined values (all using unit of (angstrom)²), and the Pearson's product-moment correlation coefficient and the Spearman's rank correlation coefficient with the average number of visually detectable particles per syringe of the sample having the air volume of 120 µL shown in Table 2 of Example 1 are shown in Table 3.

As a result, it was found that there was a high correlation between the value of Patch_ion and the average number of visually detectable particles per syringe of the sample having the air volume of 120 µL (product-moment correlation coefficient: 0.91). Besides, it was found that a sum of "Patch_hyd_5" and "(Patch_ion*1.5)" had a highest correlation with the average number of visually detectable particles per syringe of the sample having the air volume of 120 µL (product-moment correlation coefficient: 0.92).

**[Table 3]**

| Table 3 Calculated Values by MOE 2019.01 (unit: (angstrom)²) of Six Antibodies (mAb1 to mAb6), and Correlation Coefficients with Average of Visually Detectable Particles per Syringe of Sample having Air Volume of 120 µL | | | | | |
|---|---|---|---|---|---|
| Sample Name | Patch_hyd_5 | Patch_hyd | Patch_ion_5 | Patch_ion | Patch_hyd_5 +(Patch_ion*1. 5) |
| mAb6 | 690 | 2310 | 500 | 4040 | 6750 |
| mAb4 | 720 | 2720 | 550 | 2660 | 4710 |
| mAb2 | 710 | 2560 | 500 | 2420 | 4340 |
| mAb3 | 860 | 2400 | 300 | 620 | 1790 |
| mAb5 | 710 | 2190 | 260 | 720 | 1790 |
| mAb1 | 730 | 2720 | 220 | 580 | 1600 |
| Product-moment Correlation Coefficient | <0.80 | <0.80 | <0.80 | 0.91 | 0.92 |
| Rank Correlation Coefficient | <0.80 | <0.80 | <0.80 | 0.94 | 0.99 |

A relationship between the average number of visually detectable particles per syringe of the sample having the air volume of 120 µL shown in Table 2 of Example 1 and the value of "Patch_hyd_5 + (Patch_ion*1.5)" is shown in Table 4 below. By comparing the numbers of visually detectable particles in the samples having the air volumes of 120 µL and 10 µL, a degree of reduction of the average number of visually detectable particles (reduction rate of visually detectable particles) was provided.

**[Table 4]**

| Table 4 Relationship between Average of Visually Detectable Particles per Syringe and "Patch_hyd_5+(Patch_ion*1.5)" | | | | |
|---|---|---|---|---|
| Sample Name | Average of Visually Detectable Particles per Syringe | | Reduction Rate of Visually Detectable Particles (%) | Patch_hyd_5 +(Patch_ion*1.5) |
| | Air Volume 120 µL | Air Volume 10 µL | | |
| mAb6 | 5.0 | 2.0 | 60 | 6750 |
| mAb4 | 2.2 | 0.5 | 77 | 4710 |
| mAb2 | 1.5 | 1.0 | 33 | 4340 |
| mAb3 | 1.3 | 0.3 | 77 | 1790 |
| mAb5 | 0.6 | 0.2 | 67 | 1790 |
| mAb1 | 0 | 0 | - | 1600 |

### Example 3 Confirmation test for formation of visually detectable particles of mAb2

An mAb2-containing solution (mAb2: 150 mg/mL, buffer: 20 mmol/L histidine, stabilizer: 150 mmol/L arginine and about 162 mmol/L aspartic acid, surfactant: 0.5 mg/mL poloxamer 188, pH 6.0) was filtrated with a 0.22 µm filter, then, 1.0 mL of the resultant solution was filled in a COP syringe with a 27G needle (1 mL standard) having been sterilized with radiation (25 kGy), and the resultant syringe was stoppered with a stopper. With respect to the filled and stoppered sample, Evaluation 1 of visually detectable particles was performed immediately after stoppering, and a sample determined to contain visually detectable particles in the syringe was excluded. Based on the standard curve created in Example 1, the position of the stopper of each syringe was adjusted to be adjusted to a target air volume shown in Table 5. Each sample of each air volume was stored at 5°C for about 7 months, then the storage was changed to 25°C storage, and it was stored at 25°C for 6 weeks. During the 25°C storage, mechanical stress described below was applied three times, and Evaluation 2 of visually detectable particles was performed after elapse of 6 weeks. In a sample found to contain visually detectable particles present therein, the visually detectable particles were identified by Raman spectrum measurement using Raman Imaging Microscope (DXR2xi) to confirm that the particles were derived from mAb2.

### [Evaluation 1 of visually detectable particles]

It was examined whether or not visually detectable particles were present in the solution filled in the syringe in the same manner as in Evaluation 1 of visually detectable particles of Example 1 except that the brightness at the position directly below a white light source was about 8,000 lx.

**[Table 5]**

| Table 5 Setting Conditions for Air Volume | |
|---|---|
| Distance from Flange to Stopper | Air Volume |
| 11 mm | 120 µL |
| 12 mm | 90 µL |
| 12.7 mm | 69 µL |
| 13.5 mm | 46 µL |
| 14.3 mm | 23 µL |
| 15.1 mm | 3 µL |

### [Mechanical stress]

Referring to ASTM D4169, the following drop test and vibration test were combined in the order of the drop test, the vibration test, and the drop test for stress application.

### [Drop test]

A pre-filled syringe was put in a tray, and total three trays were stacked. The trays were stacked in the descending order of an empty tray, a sample tray, and an empty tray. Surfaces of a cardboard box were numbered as illustrated in Figure 2. The stacked trays were packaged in the cardboard box with the needle tip of the pre-filled syringe facing the surface 2. The sample thus packaged in the cardboard box was dropped from a height of 38.1 cm with the surface facing downward in the drop changed in the order of the surface 1, the surface 2, the surface 3 and the surface 4. With such drop defined as one set of the drop, two sets of the drop were performed in one drop test.

### [Vibration test]

A syringe sample was put in a tray, the tray was packaged in a cardboard box, and the cardboard box was placed with the barrel of the syringe extending in parallel to the ground. Vibration stress was applied to the cardboard box with intensity set to Truck Low 40 min, Truck Middle 15 min, Truck High 5 min, and Air level I 120 min.

### [Evaluation 2 of visually detectable particles]

It was examined whether or not visually detectable particles were present in the solution filled in the syringe in the same manner as in Evaluation 1 of visually detectable particles of Example 1 except that the brightness at the position directly below a white light source was about 6,000 lx.

### [Method for identifying visually detectable particles]

In each of all samples that had been found to contain visually detectable particles by Evaluation 2 of visually detectable particles performed after the mechanical stress application, the entire amount of the solution was aspiration filtered with a nickel filter having a pore size of 3 µm. A Raman spectrum of a foreign matter having the largest size among particles captured on the filter was obtained, and it was confirmed by identification that the particle was derived from mAb2.

### [Evaluation results]

Results of identification of the visually detectable particles obtained after the mechanical stress application are shown in Table 6 below. As shown below, even when an appropriate amount of a surfactant was contained, visually detectable proteinaceous particles were found in a plurality of samples when the air volume was usual air volume of 120 µL. It was found that when the air volume was reduced to 69 µL or less, visually detectable proteinaceous particles, which could not be inhibited by addition of a surfactant, could be reduced.

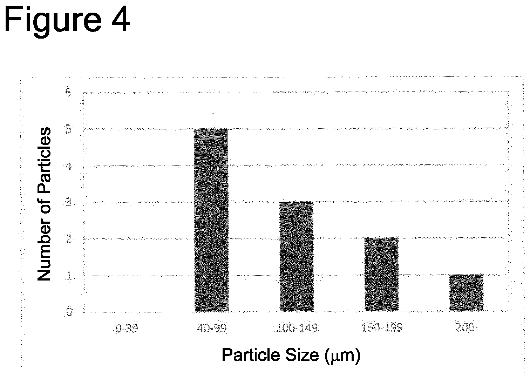

A histogram of sizes of the visually detectable proteinaceous particles thus identified is illustrated in Figure 4. A range of the sizes of the visually detectable proteinaceous particles was 46.0 to 279 µm.

**[Table 6]**

| Table 6 Results of Identification of Visually Detectable Particles after Mechanical Stress Application | | | | |
|---|---|---|---|---|
| Sample Name | Filled Amount (mL) | Air Volume (µL) | Number of Samples | Number of Samples Containing Visually Detectable Proteinaceous Particles |
| mAb2-01 | 1 | 120 | 10 | 4 |
| mAb2-02 | | 90 | 10 | 4 |
| mAb2-03 | | 69 | 10 | 1 |
| mAb2-04 | | 46 | 10 | 1 |
| mAb2-05 | | 23 | 10 | 1 |
| mAb2-06 | | 3 | 10 | 0 |

### Example 4 Confirmation test for formation of visible particles of mAb3

An mAb3-containing solution (mAb3: 120 mg/mL, buffer: 20 mmol/L histidine, stabilizer: 150 mmol/L arginine and about 162 mmol/L aspartic acid, surfactant: 0.5 mg/mL poloxamer 188, pH 6.0) was filtrated with a 0.22 µm filter, then, 1.0 mL of the resultant solution was filled in a COP syringe with a 27G needle (1 mL standard) having been sterilized with radiation (25 kGy), and the resultant syringe was stoppered with a stopper. With respect to the filled and stoppered sample, Evaluation 1 of visible particles was performed immediately after stoppering, and a sample determined to contain visible particles in the syringe was excluded. Based on the standard curve created in Example 1, the position of the stopper of the syringe was adjusted to be adjusted to a target air volume shown in Table 7. Each sample of each air volume was stored at 40°C for about 60 days after mechanical stress application at the beginning of the test, and then, Evaluation 2 of visible particles was performed under the same conditions as in Evaluation 1 of visible particles of the present example.

### [Evaluation 1 of visible particles]

In the same manner as in Evaluation 1 of visually detectable particles of Example 1, the syringe was slowly rotated or inverted at a brightness of about 3,000 to 3,750 lx, in front of a black background for 11 seconds or longer and in front of a white background for 5 seconds or longer, and was observed to examine whether or not visible particles were present in the solution filled in the syringe.

**[Table 7]**

| Table 7 Setting Conditions for Air Volume | |
|---|---|
| Distance from Flange to Stopper | Air Volume |
| 11 mm | 120 µL |
| 12.5 mm | 76 µL |
| 13.7 mm | 42 µL |
| 15.1 mm | 3 µL |

### [Mechanical stress]

Referring to ASTM D4169, vibration stress was applied under the following conditions. Then, rotation stress was applied 200 times under the following conditions.

### [Vibration stress]

A syringe sample was put in a tub, and the tub was placed with the barrel of the syringe extending vertically to the ground. Vibration stress was applied to the tub with intensity of Truck Low 40 min, Truck Middle 15 min, Truck High 5 min, and Air level II 120 min.

### [Rotation stress]

A syringe sample was put in a tub to be placed with the barrel of the syringe extending vertically to the ground. The resultant was manually rotated at a speed at which the air within the syringe sufficiently moved.

### [Evaluation results]

Results of Evaluation 2 of visible particles obtained after storage at 40°C for 60 days are shown in Table 8 below. In the same manner as in Table 6 of Example 3, even when an appropriate amount of a surfactant was contained, visible particles were found in a plurality of samples having the air volume of 120 µL. It was found that when the air volume was reduced to 42 µL or less, the number of visible particles, which could not be inhibited by addition of a surfactant, could be reduced.

**[Table 8]**

| Table 8 Evaluation Results of Visible Particles after Storage at 40°C for 60 days | | | | |
|---|---|---|---|---|
| Sample Name | Filled Amount (mL) | Air Volume (µL) | Number of Samples | Number of Samples Containing Visually Detectable Particles |
| mAb3-01 | 1 | 120 | 20 | 3 |
| mAb3-02 | | 76 | 20 | 3 |
| mAb3-03 | | 42 | 20 | 1 |
| mAb3-04 | | 3 | 20 | 0 |

### Example 5 Confirmation test for formation of visually detectable particles of mAb3

An mAb3-containing solution (mAb3: 120 mg/mL, buffer: 20 mmol/L histidine, stabilizer: 150 mmol/L arginine and 162 mmol/L aspartic acid, surfactant: 0.5 mg/mL poloxamer 188, pH 6.0) was filtrated with a 0.22 µm filter, then, 2.0 mL of the resultant solution was filled in a COP syringe with a 27G needle (2.25 mL standard) having been sterilized with radiation (25 kGy), and the resultant syringe was stoppered with a stopper. With respect to the filled and stoppered sample, Evaluation 1 of visually detectable particles was performed immediately after stoppering, and a sample determined to contain visually detectable particles in the syringe was excluded. Based on a standard curve created by the following method for measuring/setting an air volume, the position of the stopper of the syringe was adjusted to be adjusted to a target air volume shown in Table 9. Each sample of each air volume was stored at 25°C for about 3 months, and then Evaluation 2 of visually detectable particles was performed. During the storage, mechanical stress was applied three times in total, that is, at the beginning of the storage, 2 weeks after starting the storage, and 3 weeks after starting the storage.

### [Evaluation 1 of visually detectable particles]

It was examined whether or not visually detectable particles were present in the solution filled in the syringe in the same manner as in Evaluation 1 of visually detectable particles of Example 1 except that the brightness at the position directly below a white light source was about 8,000 lx.

### [Method for measuring/setting air volume]

A COP syringe with a 27G needle (2.25 mL standard) of each antibody solution-containing syringe sample filled with 2.0 mL of the antibody-containing solution and stoppered with a stopper was held with the needle facing upward, air was pushed out from the needle tip by ascending the air up to the base of the needle, and thus, a sample from which air was let out as much as possible was prepared. A distance from the flange to the stopper was 14.2 mm.

Into the sample from which air had been let out as much as possible, 120 µL of air was injected by sticking a needle of another syringe from a rubber stopper side of the sample. The distance from the flange to the stopper was 12 mm.

Based on these measurement results, a standard curve of the distance from the flange to the stopper and the air volume was created, and the air volume was set in accordance with the distance from the flange to the stopper. The conditions for the air volume thus set are shown in Table 9 below.

**[Table 9]**

| Table 9 Setting Conditions for Air Volume | |
|---|---|
| Distance from Flange to Stopper | Air Volume |
| 12 mm | 120 µL |
| 14.02 mm | 10 µL |
| 14.2 mm | 3 µL ⁻ |

### [Mechanical stress]

Referring to ASTM D4169, the following drop test and vibration test were combined in the order of the drop test, the vibration test, and the drop test for stress application at the beginning of storage. Stress of the drop test alone was applied by repeating 2 sets of the drop test in two drop tests performed 2 weeks after starting the storage and 3 weeks after starting the storage. The drop test and the vibration test were performed in the same manner as in Example 3.

### [Evaluation 2 of visually detectable particles]

It was examined whether or not visually detectable particles were present in the solution filled in the syringe in the same manner as in Evaluation 1 of visually detectable particles of Example 1 except that the brightness at the position directly below a white light source was about 6,000 lx.

### [Evaluation results]

Results of the evaluation of visually detectable particles obtained after storage at 25°C for about 3 months are shown in Table 8 below. In the same manner as in Table 6 of Example 3, even when an appropriate amount of a surfactant was contained, visually detectable particles were found to be present in a plurality of samples having the air volume of 120 µL.

It was found that when the air volume was reduced to 10 µL or less, the number of visually detectable particles, which could not be inhibited by addition of a surfactant, could be reduced.

**[Table 10]**

| Table 10 Evaluation Results of Visually Detectable Particles after Storage at 25°C for 3 Months | | | | |
|---|---|---|---|---|
| Sample Name | Filled Amount (mL) | Air Volume (µL) | Number of Samples | Number of Samples Containing Visually Detectable Particles |
| mAb3-01 | 2 | 120 | 10 | 10 |
| mAb3-02 | | 10 | 10 | 3 |
| mAb3-03 | | 3 | 10 | 1 |

## Claims

1. A method for determining, in a pharmaceutical formulation comprising a protein as an active ingredient in a solution, a protein having a high risk of forming particles in a solution, the method comprising:
constructing a three-dimensional structure model of a protein based on an amino acid sequence of the protein by homology modeling or antibody modeling;
specifying portions, on a surface of the obtained model, corresponding to a cluster of hydrophobic residues and corresponding to a cluster of residues with a charge as a hydrophobic patch and a charged patch, respectively, and calculating areas of the patches; calculating a sum of areas of top 5 hydrophobic patches ranked according to the area (X ((angstrom)²)), and a total area of charged patches (Y ((angstrom)²)); and
determining that a protein having a "X + Y × 1.5" value of 1,700 or greater is a protein having a high risk of forming particles in a solution,
wherein the particles have a particle size of 40 µm or greater.

2. The method according to claim 1, wherein a protein having a "X + Y × 1.5" value of 2,000 or greater is determined as a protein having a high risk of forming particles in a solution.

3. A method for determining, in a pharmaceutical formulation comprising a protein as an active ingredient in a solution, a protein having a high risk of forming particles in a solution, the method comprising:
constructing a three-dimensional structure model of a protein based on an amino acid sequence of the protein by homology modeling or antibody modeling;
specifying a portion, on a surface of the obtained model, corresponding to a cluster of residues with a charge as a charged patch, and calculating a total area of charged patches (Y ((angstrom)²); and
determining that a protein having a Y value of 600 or greater is a protein having a high risk of forming particles in a solution,
wherein the particles have a particle size of 40 µm or greater.

4. The method according to any one of claims 1 to 3, wherein the solution is an aqueous solution.

5. The method according to any one of claims 1 to 4, wherein the protein is a monoclonal antibody.

6. The method according to claim 5, wherein the monoclonal antibody is a monospecific antibody or a bispecific antibody.

7. The method according to any one of claims 1 to 6, wherein the homology modeling or the antibody modeling is performed using Molecular Operating Environment (MOE) software.

8. A method for reducing, in a formulation for injection in which a solution comprising a protein as an active ingredient is filled in a container, formation of particles in a solution, the method comprising:
reducing a volume of air bubbles in the container to 40 µL or less,
wherein the container is a syringe or a cartridge, and
the protein is the protein determined, by the method according to any one of claims 1 to 7, to have a high risk of forming particles in a solution.

9. The method according to claim 8, comprising reducing the volume of the air bubbles in the container to 10 µL or less.

10. A method for preparing a formulation for injection in which a solution comprising a protein as an active ingredient is filled in a container, comprising:
filling the solution in the container in such a manner as to have a volume of air bubbles of 40 µL or less in the container in the formulation for injection to be obtained,
wherein the container is a syringe or a cartridge, and
the protein is the protein determined, by the method according to any one of claims 1 to 7, to have a high risk of forming particles in a solution.

11. The method according to claim 9, comprising filling the solution in the container in such a manner as to have a volume of air bubbles of 10 µL or less in the container in the formulation for injection to be obtained.

12. The method according to claim 10 or 11, wherein the solution is an aqueous solution.

13. A formulation for injection in which a solution comprising a protein as an active ingredient is filled in a container,
wherein the protein is the protein determined, by the method according to any one of claims 1 to 6, to have a high risk of forming particles in a solution,
the container is a syringe or a cartridge, and
a volume of air bubbles in the container is 40 µL or less.

14. The formulation for injection according to claim 13, wherein the volume of air bubbles in the container is 10 µL or less.

15. The formulation for injection according to claim 13 or 14, wherein the solution is an aqueous solution.

16. A system for determining, in a pharmaceutical formulation comprising a protein as an active ingredient in a solution, a protein having a high risk of forming particles in a solution, the system comprising:
means for constructing a three-dimensional structure model of a protein based on an amino acid sequence of the protein by homology modeling or antibody modeling;
means for specifying, in a surface of the obtained model, a portion where hydrophobic residues are accumulated in a cluster, and a portion where residues with a charge are accumulated in a cluster as a hydrophobic patch and a charged patch, respectively, and calculating areas of the patches;
means for calculating a sum of areas of top 5 hydrophobic patches ranked according to the area (X ((angstrom)²)), and a total area of charged patches (Y ((angstrom)²)); and
means for determining that a protein having a "X + Y × 1.5" value of 1,700 or greater is a protein having a high risk of forming particles in a solution,
wherein the particles have a particle size of 40 µm or greater.

17. A system for determining, in a pharmaceutical formulation comprising a protein as an active ingredient in a solution, a protein having a high risk of forming particles in a solution, the system comprising:
means for constructing a three-dimensional structure model of a protein based on an amino acid sequence of the protein by homology modeling or antibody modeling;
means for specifying a portion, on a surface of the obtained model, corresponding to a cluster of residues with a charge as a charged patch, and calculating a total area of charged patches (Y ((angstrom)²)); and
means for determining that a protein having a Y value of 600 or greater is a protein having a high risk of forming particles in a solution,
wherein the particles have a particle size of 40 µm or greater.
